# EUROPÄISCHE PATENTSCHRIFT

(11) **EP 1 715 339 B1**
(45) Veröffentlichungstag und Bekanntmachung des Hinweises auf die Patenterteilung: **26.12.2012**
(21) Anmeldenummer: 06450048.1
(22) Anmeldetag: 30.03.2006
(51) Int. Cl.: G01N 25/22, F23G 5/50, G06F 19/00

(54) **Verfahren zur Ermittlung der Anteile biogener und fossiler Energieträger sowie biogener und fossiler Kohlendioxidemissionen beim Betrieb von Verbrennungsanlagen**
Method for determining the proportion of biogenic and fossil energy carriers and of biogenic and fossil carbon dioxide emissions in the operation of combustion systems
Procédé destiné à la détermination des parts de sources d'énergie biogènes et fossiles tout comme émissions de dioxyde de carbone biogènes et fossiles lors du fonctionnement d'incinérateurs

(30) Priorität: 30.03.2005 AT 5392005
(43) Veröffentlichungstag der Anmeldung: 25.10.2006
(73) Patentinhaber: Technische Universität Wien, 1040 Wien (AT)
(72) Erfinder: Fellner, Johann, 1130 Wien (AT); Cencic, Oliver, 2404 Petronell-Carnunturn (AT); Rechberger Helmut, 1070 Wien (AT)
(74) Vertreter: Patentanwälte Barger, Piso & Partner

(56) Entgegenhaltungen:
- AT-B- 409 191
- US-A- 5 367 470
- US-B1- 6 675 726
- DATABASE BIOSIS [Online] BIOSCIENCES INFORMATION SERVICE, PHILADELPHIA, PA, US; 20. Januar 2004 (2004-01-20), HUI DAFENG ET AL: "Gap-filling missing data in eddy covariance measurements using multiple imputation (MI) for annual estimations." XP002442686 Database accession no. PREV200400137643 & AGRICULTURAL AND FOREST METEOROLOGY, Bd. 121, Nr. 1-2, 20. Januar 2004 (2004-01-20), Seiten 93-111, ISSN: 0168-1923
- DATABASE COMPENDEX [Online] ENGINEERING INFORMATION, INC., NEW YORK, NY, US; 2004, YANG HAIRUI ET AL: "Model research on material balance in a circulating fluidized bed boiler" XP002442687 Database accession no. E2004428413106 & PROC. INTERNAT. SYMP. COAL COMBUST.; PROCEEDINGS OF THE 5TH INTERNATIONAL SYMPOSIUM ON COAL COMBUSTION, 2003, Seiten 251-255,

## Beschreibung

Die Erfindung betrifft ein Verfahren zur Ermittlung der Anteile biogener und fossiler Energieträger sowie biogener und fossiler Kohlendioxidemissionen beim Betrieb von Verbrennungsanlagen in Übereinstimmung mit dem Kennzeichnenden Teil des Anspruches 1 und der AT 409191 B.

Die AT 409191 B offenbart eine Bestimmungsmöglichkeit für die Relation von emeuerbaren zu nicht erneuerbaren Energieträgern in einem Brennstoffgemisch über die Isotopenverteilung zumindest eines instabilen Isotops, das im Reaktionsprodukt vorhanden ist. Durch die Instabilität des Isotops kann aus dem Verhältnis der Isotopenverteilung das Durchschnittsalter des Brennstoffes und unter Zugrundelegung des Zeitpunktes T=0 für emeuerbare Energieträger und hypothetisch T=oo für fossile Energieträger die Masseverteilung bestimmt werden. Die Bestimmung der Isotopenverteilung ist aber aufwändig und für eine ganze Reihe von Brennstoffen gilt keine der beiden Annahmen für die Zeitpunkte ihres Entstehens.

Die US 6,675,726 B hat nichts mit der Ermittlung der Anteile biogener und fossiler Energieträger zu tun, sondern die Aufgabe, auf die momentanen Verfahrenparameter der Verbrennung Rückschlüsse zu ziehen und durch entsprechende Veränderung der beeinflussbaren Anfangswerte (Änderung der Brennmaterialzufuhr, Änderung der Luftzufuhr, Änderung der Geschwindigkeit des Wanderrostes, Änderung des Verhältnisses Primärluft-Sekundärluft, etc...) darauf so zu reagieren, dass eine möglichst vollständige Verbrennung erzielt wird. Um die Verfahrenparameter zu bestimmen, wird vorgeschlagen, verschiedene Bilanzen des Verbrennungsvorganges im Zusammenhang mit Sensoren im Abgasstrom zur Bestimmung des Gehaltes von Kohlendioxid, Sauerstoff und Wasser zu verwenden.

Die US 5,367,470 A hat genau die gleichen Aufgaben wie die letztgenannte Druckschrift, allerdings für einen mit fossilen Brennstoffen betriebenen Dampferzeuger. Schon daher gibt es keinen Grund für ihre Autoren, den fossilen bzw. biogenen Anteil des Brennstoffes bestimmen zu wollen.

Die Betreiber von Müllverbrennungsanlagen bzw. Mitverbrennungsanlagen (darunter versteht man eine Verbrennungsanlage, in der Abfälle als Ersatz- oder Zusatzbrennstoff bis zu 40 % der in einem Kalendervierteljahr tatsächlich zugeführten durchschnittlichen Gesamtbrennstoffwärmeleistung eingesetzt werden) mit Stromauskoppelung sind gesetzlich verpflichtet, die während vorgegebener Zeiträume eingesetzten Primärenergieträger auszuweisen. Hintergrund ist, dass die bei der Verbrennung von Abfällen erzeugte elektrische Energie von verschiedenen Primärenergieträgern stammt. Zum einen sind dies Biomasse wie Holz, Papier, Küchen- und Gartenabfälle u.dgl., zum anderen fossile Brennstoffe, wie insbesondere Kunststoffe, die aus Erdöl hergestellt werden. Analog zur Stromkennzeichnung wird es zukünftig im Rahmen des Emissionszertifikatshandels für alle Betreiber von Müllverbrennungsanlagen bzw. Mitverbrennungsanlagen erforderlich sein, biogene und fossile Kohlendioxidemissionen auszuweisen.

Um der bestehenden (und zukünftigen) gesetzlichen Verpflichtung nachzukommen, war es im Stand der Technik bekannt, Abfallsortieranalysen zu betreiben und den einzelnen Abfallfraktionen einen biomassebürtigen bzw. einen erdölproduktbürtigen Heizwert (bzw. Kohlenstoffgehalt) zuzuordnen. Dieses Verfahren ist nicht nur kostspielig, für die durchführenden Personen äußerst unangenehm und unter Umständen gefährlich, sondern es ist auch mit großen Unsicherheiten behaftet, die einerseits auf die unsichere Bewertung der Heizwerte der einzelnen Fraktionen, andererseits auf kaum erfassbare jahreszeitliche Schwankungen in der Abfallzusammensetzung zurückgehen. Schließlich tritt auch eine allgemeine längerfristige Drift der Zusammensetzung des Abfalls durch Änderungen im Einzugsgebiet der Müllverbrennungsanlage, durch Änderungen in den Lebensgewohnheiten der Personen, deren Abfall verbrannt wird und letztlich auch durch rechtliche

Änderungen auf, die so nicht erfasst werden können. Derartige rechtliche Änderungen können beispielsweise Änderungen im Recyclingsystem sein, wenn etwa ab einem gesetzlich vorgegebenen Zeitpunkt nicht mehr aller Kunststoffinüll gesammelt und recycelt wird, sondern nur mehr PET-Flaschen, und die anderen Kunststoffe der Verbrennung zugeführt werden.

Es ist nun ein Ziel der Erfindung, ein Verfahren der eingangs genannten Art anzugeben, das die genannten Nachteile nicht aufweist und es erlaubt, genauere Werte zu erhalten bzw. auch Aussagen über längere Zeiträume machen zu können. All dies soll mit geringem Aufwand möglich sein und insbesondere den Betrieb der Verbrennungsanlage nicht beeinträchtigen.

Erfindungsgemäß wird diese Aufgabe mit einem eingangs erwähnten Verfahren dadurch gelöst, dass die Ermittlung der Anteile durch die Bestimmung von zumindest drei der folgenden sieben Bilanzen erfolgt: Der Massenbilanz, der Aschenbilanz, der Kohlenstoffbilanz, der Energiebilanz, der Sauerstoffumsatzbilanz, der Bilanz aus Sauerstoffverbrauch und Kohlendioxidproduktion und der Wasserbilanz.

Die einzelnen Bilanzen können auf einfache Weise ermittelt werden, wodurch auch jahreszeitlichen Schwankungen in der Abfallzusammensetzung Rechnung getragen werden kann. Die Einbeziehung mehrerer unterschiedlicher Bilanzen liefert sehr genaue Resultate für die Massenanteile, die Brennwertanteile bzw. für die Anteile der biogenen und fossilen Kohlendioxidemissionen.

Um die Genauigkeit der Ergebnisse zu erhöhen bzw. Fehlergrenzen exakter angeben zu können, erfolgt die Ermittlung der Brennwertanteile bzw. der Kohlendioxidemissionsanteile vorzugsweise durch die Bestimmung zumindest einer weiteren, zusätzlichen Bilanz.

In einer bevorzugten Variante der Erfindung ist das Verfahren dadurch gekennzeichnet, dass die Ermittlung der Anteile durch die Bestimmung der Massenbilanz, der Aschenbilanz, der Kohlenstoffbilanz, der Energiebilanz, der Sauerstoffumsatzbilanz und der Bilanz aus Sauerstoffverbrauch und Kohlendioxidproduktion erfolgt. Diese sechs Bilanzen können besonders gut bestimmt werden und liefern daher auch sehr genaue Daten mit geringen Fehlergrenzen.

Vorteilhafterweise ist das erfindungsgemäße Verfahren dadurch gekennzeichnet, dass die Anteile mittels Ausgleichsrechnung ermittelt werden. Die Ausgleichsrechnung stellt ein effizientes Instrument zur Bestimmung von Unbekannten in einem überbestimmten System dar.

In einer Variante ist das erfindungsgemäße Verfahren dadurch gekennzeichnet, dass die Anteile mittels Monte Carlo Simulation ermittelt werden.

Die Erfindung wird im Folgenden an Hand der theoretischen Überlegung, der Zeichnung und schließlich eines praktischen Beispiels näher erläutert, ohne darauf beschränkt zu sein.

So kann insbesondere die verwendete Anzahl bzw. Kombination von Bilanzgleichungen zur Bestimmung der Anteile variiert werden. In der Zeichnung zeigt
die Fig. 1 die graphische Gegenüberstellung der Messwerte und der Näherungswerte der Formeln nach Michel und nach Boie,
die Fig. 2 die Systemgrenze des betrachteten Systems und
die Fig. 3 bis 7 graphische Darstellungen und Erläuterungen der Berechnungen.

### Begriffsdefinitionen Energieinhalt

Der obere Heizwert Hₒ (auch Brennwert) und der unterer Heizwert Hᵤ (auch Heizwert) eines Brennstoffes sind Maße für die bei der vollständigen Verbrennung frei werdenden Wärmemengen.

Nach DIN 51900 ist der obere Heizwert (Brennwert) definiert als der Quotient aus der bei vollständiger und vollkommener Verbrennung einer bestimmten Masse eines festen oder flüssigen Brennstoffes frei werdenden Wärmemenge und der Masse des Brennstoffes, wenn die Temperatur des Brennstoffes vor dem Verbrennen und die seiner Verbrennungsrückstände 25°C beträgt, wenn das vor dem Verbrennen im Brennstoff vorhandene Wasser und das beim Verbrennen der wasserstoffhaltigen Verbindungen des Brennstoffes gebildete Wasser nach der Verbrennung im flüssigen Zustand vorliegen. Außerdem müssen die Verbrennungserzeugnisse des Kohlenstoffs und des Schwefels als Kohlendioxid und als Schwefeldioxid in gasförmigen Zustand vorliegen.

Der untere Heizwert Hᵤ unterscheidet sich vom oberen Heizwert Hₒ im Wesentlichen dadurch, dass das Wasser nach der Verbrennung im gasförmigen Zustand vorliegt, d.h. der untere Heizwert ist gegenüber dem oberen Heizwert um die Verdampfungswärme des Wassers reduziert..

### Heizwert bzw. Brennwertbestimmung

Die Bestimmung des Brennwertes eines Brennstoffs kann experimentell oder mathematisch über Näherungsgleichungen (bei Kenntnis der Elementarzusammensetzung) erfolgen. Bei der experimentellen Bestimmung wird zwischen Messungen im Labor mittels Kalorimetern und Messungen an großtechnischen Versuchsanlagen unterschieden.

Für die Bestimmung des Brenn- bzw. Heizwertes von Abfällen (üblicherweise ein Gemisch aus verschiedenen Brennstoffen) sind folgende Methoden denkbar:
- Experimentell aus den Betriebsdaten bei der Verbrennung der Abfälle: der Heizwert errechnet sich aus der Energiebilanz der Verbrennungsanlage
- Brennwertberechung anhand der Einzelfraktionen der Abfälle: der Brennwert des Abfalls ergibt sich aus den Massenanteilen der Einzelfraktionen und den dazugehörigen Brennwerten der Fraktionen ${H}_{o , ges} = {\sum }_{ } ⁢ {m}_{Br , E} ⋅ {H}_{o , E}$
   *H_{o,ges} Brennwert des Abfalls [MJ*/*kg FS]*
   *m_{Br,E} Massenanteil der Einzelfraktionen im Abfall [kg*/*kg]*
   *H_{o,E} Brennwert der Einzelfraktionen [MJ*/*kg FS]*
- Brennwertbestimmung über die Elementarzusammensetzung der Abfälle: der Brennwert des Abfalls wird mit Hilfe empirischer Formeln in die Gehalte an C, H, O, S und N einfließen, abgeschätzt.

Tabelle 1 beinhaltet eine Auswahl bekannter Formeln zu Berechnung des Energieinhaltes von Brennstoffen. Eine exakte Berechnung des Brennwertes ist mithilfe dieser Methode allerdings nicht möglich, da die chemischen Bindungsformen in den Formeln im Einzelfall nicht berücksichtigt werden. Jede Brennstoffformel gilt daher für eine gewisse Art von Brennstoffen.

**Tabelle Empirische Formeln zur Berechnung des Brenn- bzw. Heizwertes eines Brennstoffs (bei Kenntnis der Elementarzusammensetzung)**

| **Literatur** | **Brennwert bzw. Heizwert [MJ/kg FS]** |
|---|---|
| Michel (1938) | *Hₒ* = 34,889 · *c_{C}* + 124,348 · *c_{H}* -10,467 · *c_{O}* + 6,28 · *c_{N}* + 19,092 · *c_{S}* |
| Dulong (zitiert in Beiz & Küttner, 1986) | *Hₒ* = 34,827 · *c_{C}* + 144,267 · *-c_{H}* -18,0337 · *c_{O}* + 9,420 · *c_{S}* |
| Boie (1957) | *Hᵤ* = 34,834 · *c_{C}* + 93,868 · *c_{H}* -10,802 · *c_{O}* + 6,28 · *c_{N}* + 10,467 · *c_{S}* - 2,449 · *c_{H20}* |
| Verbandsformel (zitiert in Beiz & Küttner, 1986) | *Hᵤ =* 33,913 · *c_{C}* + 121,417 · *c_{H}* - 115,177 · *c_{O}* + 10,467 · *c_{S}* - 2,512 · *c_{H20}* |

| | |
|---|---|
| *c_{C}, c_{H}, co, c_{N}, c_{S}, c_{H20} Gehalt an Kohlenstoff, Wasserstoff, Sauerstoff, Stickstoff, Schwefel und Wasser des Brennstoffs [kg*/*kg FS]* *Hᵤ, Hₒ Heizwert bzw. Brennwert des Brennstoffs [MJ*/*kg FS]* | |

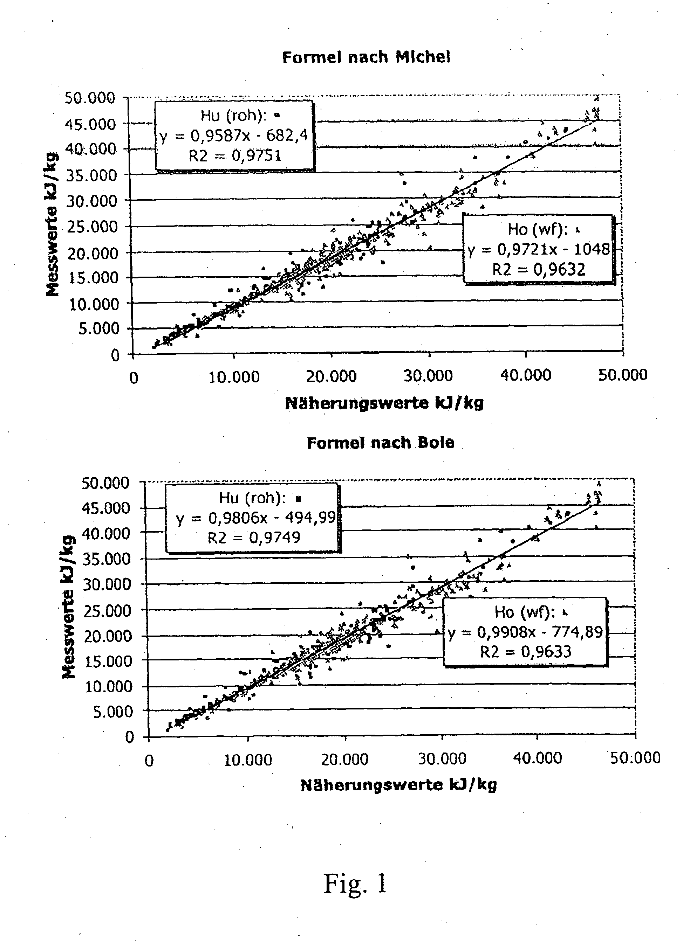

Ein Vergleich (Kost, 2001) von berechneten mit "gemessenen" Brenn- bzw. Heizwerten von Abfällen zeigt jedoch, dass mithilfe der empirischen Näherungsgleichungen der Brennwert gut abgeschätzt werden kann. Die beste Annäherung an experimentell bestimmte Heizwerte wurde mithilfe der Formeln von Boie (1957) und Michel (1938) erzielt. Allerdings kommt es auch bei Verwendung dieser Formeln zu einer generellen Überschätzung des Brenn- bzw. Heizwertes (siehe Fig. 1) von Abfällen. Dies wird durch die Verwendung von Korrekturfaktoren berücksichtigt. Bei Verwendung empirischer Nährungsgleichungen zur Heizwertbestimmung von Abfällen empfiehlt es sich daher diesen "Fehler (Überschätzung)" auszugleichen.

### Biomassebürtiger Teil des Heizwertes von Abfällen

Der Energieinhalt (Brennwert) von Abfällen (Restmüll, Gewerbemüll) beruht auf emeuerbarer organischer Substanz (Biomasse wie Holz, Papier, Küchen- und Gartenabfälle) und nicht erneuerbarer organischer Substanz (z.B. Kunststoffe). Zur Abschätzung der jeweiligen Anteile (Biomasse und fossile Energieträger) des Brennwertes am Gesamtbrennwert eignet sich prinzipiell sowohl die Brennwertbestimmung über die Einzelfraktionen als auch die Berechnung über die Elementarzusammensetzung.

Bei der Berechnung des biomassebürtigen Anteils am Heizwert über Einzelfraktionen müssen die Brennwerte der verschiedenen Fraktionen den beiden Primärenergieträgern zugeordnet werden. Dagegen sind bei der Bestimmung anhand der Elementarzusammensetzung separate Angaben über die stoffliche Zusammensetzung (C, H, S, N, O, H₂O) der Biomasse und der fossilen Energieträger im Abfall erforderlich.

### Bestehende Ansätze zur Bestimmung des biomassebürtigen Brennwertanteils

Im Stand der Technik bekannte Studien (Würdinger et al., 1997; TBHauer, 2000) zur Bestimmung des regenerativen Anteils an Primärenergieträgern bei der thermischen Verwertung von Restmüll beruhen auf der Brennwertberechnung anhand von Einzelfraktionen, wobei die Brennwerte der einzelnen Fraktionen den Primärenergieträgern zugeordnet wurden. Die Vorgangsweise dieser Methode gliedert sich im Detail in folgende Arbeitsschritte:
1. Bestimmung der Abfallzusammensetzung (Fraktionen) durch Sortieranalysen bzw. basierend auf Literaturdaten
2. Bewertung der einzelnen Abfallfraktionen mit Heizwerten
3. Zuordnung der einzelnen Heizwerte zu den beiden Primärenergieträgern Biomasse und Erdölprodukte
4. Berechnung des Gesamtheizwertes des betrachteten Abfalls
5. Berechnung der Heizwertanteile von Biomasse und Erdölprodukten des betrachteten Abfalls

Der auf diese Weise bestimmte biomassebürtige Heizwertanteil liegt je nach betrachteter Anlage und behandeltem Abfall zwischen 25 und 50 % (Würdinger et al., 1997, TBHauer, 2000, 2002). Die Unsicherheiten der Resultate, die sich bei Verwendung dieser Methode ergeben, beruhen einerseits auf der "unsicheren" Datenlage hinsichtlich der Abfallzusammensetzung und andererseits auf der eingeschränkten Verlässlichkeit von Brennwert- bzw. Heizwertangaben für die einzelnen Abfallfraktionen. Darüber hinaus ist die Zuordnung der Heizwerte zu Biomasse bzw. fossilen Energieträgern bei verschiedenen Abfallfraktionen schwierig durchzuführen und mit erheblichen Unsicherheiten behaftet (z.B.: Verbundstoffe oder Hygienewaren). Ein weiterer Punkt der wesentlich zur Unschärfe des Ergebnisses beiträgt, ist der unspezifizierbare Rest bei Abfallsortieranalysen, dem schwierig ein Heizwert zugeordnet werden kann.

Die Unsicherheiten dieser Methode zur Bestimmung des biomassebürtigen Heizwertanteils von Abfällen lassen sich durch aufwendige Sortieranalysen und anschließende Heizwertbestimmung der einzelnen Fraktionen verringern. Allerdings können selbst bei Durchführung aufwendiger Sortier- und Heizwertanalysen des in der betrachteten Anlage behandelten Abfalls, jahreszeitliche Schwankungen der Abfallzusammensetzung kaum quantifiziert und berücksichtigt werden. Untersuchungen von Morf et al. (2004) zeigen allerdings, dass mit starken zeitlichen Schwankungen der Abfallzusammensetzung zu rechnen ist. Es ist daher davon auszugehen, dass durch einzelne Sortieranalysen nur sehr ungenaue Resultate erzielt werden können.

### Bestehende Ansätze zur Bestimmung des biogenen und fossilen Kohlenstoffgehalts bzw. der Kohlendioxidemissionen

Die Bestimmung der "biogenen" bzw. "fossilen" Kohlendioxidemissionen bei der Müllverbrennung erfolgte bisher analog zu den oben beschriebenen Verfahren für die Brennwertanteile:
1. Bestimmung der Abfallzusammensetzung (Fraktionen) durch Sortieranalysen bzw. basierend auf Literaturdaten
2. Bewertung der einzelnen Abfallfraktionen mit Kohlenstoffgehalten
3. Zuordnung der einzelnen Kohlenstoffgehalte hinsichtlich ihres Ursprungs (biogen oder fossil)
4. Berechnung des gesamten Kohlenstoffgehalts des betrachteten Abfalls
5. Berechnung der Anteile des Kohlenstoffgehalts von Biomasse und Erdölprodukten des betrachteten Abfalls
6. Berechnung der "biogenen" und "fossilen" Kohlendioxidemissionen

Alternativ dazu kann die sogenannte C14-Methode angewandt werden: Die WO 02/06730 A1 offenbart ein Verfahren zur Bestimmung des Verhältnisses von emeuerbaren zu nicht erneuerbaren Energieträgern in einem Brennstoffgemisch, bei dem der Anteil instabiler radioaktiver Elemente, wie z.B. C14, bestimmt wird. Während der C14-Gehalt bei nicht erneuerbaren Energieträgern wie Öl, Gas und Kohle verschwindend klein und daher vernachlässigbar ist, weisen erneuerbare kohlenstoffhältige Energieträger wie Holz ein geringes Alter und daher messbare Anteile von C14 auf. Die Messung des Isotopenverhältnisses erfolgt beispielsweise im Abgasstrom über das CO₂. Das Isotopenverhältnis bzw. der Anteil von C14 wird entweder direkt mit Massenspekrometern ermittelt oder nach einer Umwandlung von CO₂ mittels Proportional- oder Flüssigkeitszählrohren. Diese Methode erfordert jedoch eine Vielzahl von kostspieligen und empfindlichen Messgeräten, mit denen die Verbrennungsanlage nachträglich ausgestattet werden muss und die einer regelmäßigen Wartung bedürfen.

**Erfindungsgemäße Methode zur Bestimmung des biomassebürtigen Brennwertanteils bzw. der biomassebürtigen Kohlendioxidemissionen bei der Verbrennung** von **Abfällen**

Aufgrund der angeführten Mängel bisheriger Studien bzw. Verfahren zur Bestimmung des biomassebürtigen Anteils am Heizwert von Abfällen (bzw. der biomassenbürtigen Kohlendioxidemissionen) wurde diese neue Methode entwickelt. Ein Ziel dieser Methode ist es, jahreszeitliche Änderungen der Müllzusammensetzung (z.B.: höherer Strauchschnittanteil im Sommer) erfassen zu können und bei der Berechnung zu berücksichtigen. Die Erfindung basiert auf der Methode der Heizwertbestimmung mittels Elementarzusammensetzung. Es wird dabei die Kenntnis der stofflichen Zusammensetzung von Biomasse und fossilen Energieträgern genützt, um den biomassebürtigen Heizwertanteil der behandelten Abfälle abschätzen zu können. Die Ergebnisse bzw. Eingangsdaten der angestellten Berechnungen werden mit den Betriebsparametern (z.B.: CO₂-Konzentration im Reingas, Reingasmenge, Dampfmenge,...) und verschiedenen Monitoringgrößen (z.B.: Menge an Schlacke,...) der Verbrennungsanlagen abgeglichen. Diese Vorgangsweise ermöglicht es, dass jahreszeitliche Änderungen in der Abfallzusammensetzung in der Berechnung Berücksichtigung finden.

Im Konkreten stützt sich die neue Methode auf die oben genannten sieben Bilanzgleichungen, die "theoretisch" hergeleitet sind. Die Ergebnisse dieser Gleichungen sind mit den auf der Verbrennungsanlage gemessenen Güter-, Stoff- und Energiebilanzen in Übereinstimmung zu bringen.

In einer ersten Variante werden für die "theoretischen" Bilanzgleichungen die unterschiedlichen Materialien des Abfalls in drei "Stoffgruppen": Inert (z.B.: Glas, Metalle), Biogen (z.B.: Papier, Küchenabfälle, ...) und Fossil (z.B.: Kunststoff, bzw. Kunststoffanteile in Textilien, Hygieneartikeln, ...) unterteilt. Diese drei Gruppen bzw. deren Massenanteile stellen die unbekannten Größen dar, die es zu bestimmen gilt. In weiterer Folge kann bei Kenntnis dieser Größen und der Elementarzusammensetzung der drei Gruppen der biomassebürtige Anteil des Heizwertes (bzw. die biomassenbürtigen Kohlendioxidemissionen) berechnet werden.

In einer zweiten Variante des Verfahrens werden die Materialen des Abfalls in vier "Stoffgruppen": Inert (nicht brennbares), Biogen (brennbare biogene Substanz), Fossil (brennbare fossile Substanz) und Wasser unterteilt. Diese Variante benötigt nur Vorinformation über die mittleren Stoffeigenschaften (C-, H-, O-, N- und S-Gehalt) der biogenen und fossilen Materialien.

Die Vorteile des vier-Gruppen-Verfahrens gegenüber dem drei-Gruppen-Verfahren sind: eine geringere Anzahl an benötigten Stoffdaten (es werden keine Vorinformationen über Wasser- und Aschegehalt der einzelnen Stoffgruppen benötigt), eine dadurch bedingte einfachere Anwendung sowie ein üblicherweise mit geringerer Unsicherheit behaftetes Ergebnis. Bei Anwendung des vier-Gruppen-Verfahrens werden jedoch für den seltenen Fall, dass die Berechnung des gesamten Massenanteils an biogenen bzw. fossilen Materialien (jeweils inklusive Wasser- und Aschegehalt) gewünscht ist, Informationen über Wasser- und Aschegehalt dieser Stoffgruppen benötigt.

### Theoretische Güter-, Stoff-, und Energiebilanzen für die Variante mit 3 Stoffgruppen

Sämtliche Einheiten sowie Erklärungen der verwendeten Formelzeichen sind am Ende der Beschreibung zusammengefasst.

### 1) "Massenbilanz":

Die Gesamtmasse ergibt sich aus der Summe der Masse an inertem Material M_{I}, Masse an biogenem Material M_{B} und Masse an fossilem Material M_{F}. Bezogen auf die gesamte Abfallmasse M_{Abfall} ergibt sich folgende Gleichung: ${m}_{I} + {m}_{B} + {m}_{F} = 1$ ${m}_{I} = \frac{{M}_{I}}{{M}_{Abfall}} {m}_{B} = \frac{{M}_{B}}{{M}_{Abfall}} {m}_{F} = \frac{{M}_{F}}{{M}_{Abfall}}$

### 2) "Aschen-Bilanz":

Der Aschegehalt des Abfalls errechnet sich aus den Aschegehalten der drei Gruppen Inert, Biogen und Fossil. Wertebereiche für die Aschengehalte dieser drei Fraktionen lassen sich wiederum aus Sortieranalysen und Literaturangaben ableiten. Der "theoretisch" errechnete Aschegehalt a_{A} des Abfalls muss mit den Schlacken- und Aschenmengen, die bei der Verbrennung anfallen, übereinstimmen. Angestellte Berechnungen zur Massenänderung von "inerten" Materialien durch beispielsweise die Oxidation von Metallen (Aluminium) zeigen, dass diese Oxidationsprozesse für die Aschebilanz unerheblich sind. Ebenso ist der Gehalt an Rauchgasreinigungsmitteln (CaO bzw. NaO) in den Rückständen (Aschen) für die Bilanz unbedeutend. ${m}_{I} ⋅ {a}_{I} ⋅ \left(1-{w}_{I}\right) + {m}_{B} ⋅ {a}_{B} ⋅ \left(1-{w}_{B}\right) + {m}_{F} ⋅ {a}_{F} ⋅ \left(1-{w}_{F}\right) = {a}_{A} ⋅ \left(1-{w}_{A}\right)$

### 3) "Kohlenstoffbilanz":

Der Kohlenstoffgehalt des Abfalls c_{CA} ergibt sich aus den einzelnen Kohlenstoffgehalten der drei Fraktionen Inert, Biogen und Fossil. Wertebereiche für die Kohlenstoffgehalte dieser drei Fraktionen lassen sich wiederum aus Sortieranalysen und Literaturangaben ableiten. Dieser rechnerisch abgeleitete Kohlenstoffgehalt des Abfalls muss mit dem mittleren Kohlenstoffgehalt des Abfalls c_{cA} übereinstimmen, der sich aus der Reingasmenge, der CO₂ Konzentration und dem Kohlenstoff-Transferkoeffizienten im Reingas T_{C,R} ermitteln lässt (z.B.: Morf et al., 1997). Um nur den tatsächlich zu CO₂ umgesetzten (verbrannten) organischen Kohlenstoff zu berücksichtigen, kann der Transferkoeffizient mit 1 angenommen werden. ${m}_{B} ⋅ {c}_{{C}_{B}} ⋅ \left(1-{w}_{B}\right) ⋅ \left(1-{a}_{B}\right) + {m}_{F} ⋅ {c}_{{C}_{F}} ⋅ \left(1-{w}_{F}\right) ⋅ \left(1-{a}_{F}\right) = {c}_{{C}_{A}}$

### 4) "Energie-Bilanz":

$\begin{matrix}{m}_{I} ⋅ \left(-2,449⋅{w}_{I}\right) + {m}_{B} ⋅ \left(\left(\begin{matrix}34 , 834 ⁢ {c}_{{C}_{B}} + 93 , 868 ⋅ {c}_{{H}_{B}} - 10 , 802 ⋅ {c}_{{O}_{B}} + \\ + 6 , 28 ⋅ {c}_{{N}_{B}} + 10 , 467 ⋅ {c}_{{S}_{B}}\end{matrix}\right)+\left(1-{w}_{B}\right)⋅\left(1-{a}_{B}\right)-2,449⋅{w}_{B}\right) + \\ + {m}_{F} ⋅ \left(\left(\begin{matrix}34 , 834 ⁢ {c}_{{C}_{F}} + 93 , 868 ⋅ {c}_{{H}_{F}} - 10 , 802 ⋅ {c}_{{O}_{F}} + \\ + 6 , 28 ⋅ {c}_{{N}_{F}} + 10 , 467 ⋅ {c}_{{S}_{F}}\end{matrix}\right)+\left(1-{w}_{F}\right)⋅\left(1-{a}_{F}\right)-2,449⋅{w}_{F}\right) = {H}_{u , A , B}\end{matrix}$

Der untere Heizwert des Abfalls kann über die Elementarzusammensetzung der drei Fraktionen Inert, Biogen und Fossil bestimmt werden, wobei dafür unterschiedliche empirische Gleichungen (siehe Tabelle 1) herangezogen werden können. Im Konkreten wird der Formel von Boie (1957), Fig. 1 der Vorzug gegeben, da bei Verwendung dieser Formel die "geringsten Korrekturen" für eine Übereinstimmung zwischen gemessenen und berechneten Heizwerten von Abfällen notiert wurde (Kost, 2001). Die berücksichtigende Korrekturgleichung für den Heizwert nach Boie lautet gemäß den Ergebnissen von Kost (2001) folgendermaßen: ${H}_{u , A} = 0 , 9806 ⋅ {H}_{u , A , B} - 0 , 495$

Dieser "rechnerisch" ermittelte Heizwert (abgeleitet aus der Elementarzusammensetzung und vermindert gemäß Kost, 2001) muss mit dem Heizwert des Abfalls übereinstimmen, der sich aus den Betriebsdaten der MVA (Dampfmenge, Wirkungsgrade, Dampfdruck, Dampftemperatur, Temperatur bzw. Druck im Kondensator) errechnen lässt.

Statt der Formel von Boie samt Korrekturgleichung kann jede andere Heizwertformel (basierend auf der Elementarzusammensetzung des Brennstoffs, z.B. siehe Tabelle 1) in Zukunft herangezogen werden, wenn sie der Realität besser entspricht als diese.

### 5) "Sauerstoffumsatzbilanz"

Der Sauerstoffumsatz der Abfallverbrennung errechnet sich aus der Elementarzusammensetzung folgendermaßen: $\begin{matrix}{m}_{B} ⋅ \left(1-{w}_{B}\right) ⋅ \left(1-{a}_{B}\right) ⋅ \left(\frac{{c}_{{C}_{B}}}{12}+\frac{{c}_{{H}_{B}}}{4}-\frac{{c}_{{O}_{B}}}{32}+\frac{{c}_{{N}_{B}}}{14}+\frac{{c}_{{S}_{B}}}{32}\right) ⋅ 1000 + \\ + {m}_{F} ⋅ \left(1-{w}_{F}\right) ⋅ \left(1-{a}_{F}\right) ⋅ \left(\frac{{c}_{{C}_{F}}}{12}+\frac{{c}_{{H}_{F}}}{4}-\frac{{c}_{{O}_{F}}}{32}+\frac{{c}_{{N}_{F}}}{14}+\frac{{c}_{{S}_{F}}}{32}\right) ⋅ 1000 = {O}_{2} ⁢ V\end{matrix}$

Der Sauerstoffumsatz infolge Oxidation anorganischer Materialien ist vernachlässigbar.

### 6) "Sauerstoffverbrauchs- und Kohlendioxidproduktions"-Bilanz

Ein eindeutiger Unterschied zwischen der Verbrennung von biogenen und fossilen Materialien findet sich im Verhältnis von O₂-Verbrauch und CO₂-Produktion.

Bei der Verbrennung von Biomasse (hier angenommen: Zellulose) werden pro Glukoseeinheit 6 Moleküle O₂ verbraucht und dabei 6 Moleküle CO₂ produziert. Die Molsumme aus Sauerstoff und Kohlendioxid in der Zuluft ist damit gleich derjenigen im Rauchgas.

(*C*₆*H*₁₀*O*₅)*ₙ +*6*O*₂ → 6*CO*₂ + 5*H*₂*O*

Bei der Verbrennung von Kunststoffen (hier angenommen: Polyethylen) werden pro Ethyleneinheit 3 Moleküle O₂ verbraucht und dabei 2 Moleküle CO₂ produziert. Der restliche Sauerstoff wird zu Oxidation des Wasserstoffs benötigt. Die Molsumme aus Sauerstoff und Kohlendioxid in der Luft vor der Verbrennung ist damit größer als nach der Verbrennung. ${\left(-{CH}_{2}-{CH}_{2}-\right)}_{n} + 3 ⁢ {O}_{2} \to 2 ⁢ {CO}_{2} + 2 ⁢ {H}_{2} ⁢ O$

Dieser Unterschied in der Zusammensetzung des Rauchgases kann zur Bestimmung des Anteils an Biomasse bzw. fossilen Materialien im behandelten Abfall verwendet werden.

In Gleichungsform ausgedrückt ergibt sich für das beschriebene Phänomen folgende Schreibweise:

Wird für die Elementargehalte der Stoffgruppen die Einheit "kg/kg aschefrei" verwendet, so sind die beiden Terme mit jeweils 1000 zu multiplizieren, um die "Sauerstoffverbrauchs-Kohlendioxidproduktions-Bilanz" in mol/kg FS" zu erhalten. $\begin{matrix}{m}_{B} ⋅ \left(1-{w}_{B}\right) ⋅ \left(1-{a}_{B}\right) ⋅ \left(\frac{{c}_{{H}_{B}}}{4}-\frac{{c}_{{O}_{B}}}{32}+\frac{{c}_{{N}_{B}}}{14}+\frac{{c}_{{S}_{B}}}{32}\right) ⋅ 1000 + \\ + {m}_{F} ⋅ \left(1-{w}_{F}\right) ⋅ \left(1-{a}_{F}\right) ⋅ \left(\frac{{c}_{{H}_{F}}}{12}-\frac{{c}_{{O}_{F}}}{32}+\frac{{c}_{{N}_{F}}}{14}+\frac{{c}_{{S}_{F}}}{32}\right) ⋅ 1000 = {Δ}_{O ⁢ 2 + CO ⁢ 2 , g}\end{matrix}$

In obiger Gleichung wurde durch die Vernachlässigung des Inertanteils angenommen, dass die inerte Stoffgruppe die selbe Menge an Sauerstoff verbraucht wie sie Kohlendioxid produziert. Diese Annahme lässt sich durch die folgenden Abschätzungen begründen: Durch das Brennen von CaCO₃ zu CaO kann aus inerten Materialien CO₂ entstehen.

*CaCO*₃ → *CaO* + *CO*₂

Bei einem mittleren Gehalt an CaCO₃ von 60 g/kg TS (berechnet nach Döberl et al., 2002) ergibt sich unter der Annahme, dass das Calciumcarbonat zu 50 % zu Calciumoxid gebrannt wird, eine CO₂-Produktion von 0,3 mol/kg TS.

Sauerstoff wird bei "inerten Stoffen" zur Oxidation von Metallen verbraucht. Die Hauptreaktion ist dabei die Oxidation von metallisch vorliegendem Aluminium zu Aluminiumoxid Al₂O₃.

4 *Al* + 3*O*₂ → 2*Al*₂*O*₃

Bei einem mittleren Aluminiumgehalt von 20 g/kg TS (Skutan & Brunner, 2003) ergibt sich unter der Annahme, dass 50 % davon als metallisches Aluminium vorliegen und dieses vollständig zu Aluminiumoxid oxidiert wird, ein Sauerstoffverbrauch von 0,28 mol/kg TS.

Der Verbrauch von O₂ bzw. die Produktion von CO₂ und damit auch die Differenz dieser beiden Werte sind für inerte (anorganische) Stoffe im Vergleich zu den organischen Materialien (Biomasse oder Kunststoffe) vernachlässigbar. Für Biomasse etwa liegt die Differenz aus Sauerstoffverbrauch und Kohlendioxidproduktion bei rund 3 mol/kg TS und bei Kunststoffen bei ungefähr 18 mol/kg TS.

### 7) "Wasserbilanz":

Ausgehend von den drei "Stoffgruppen" ergibt sich der Wassergehalt des Abfalls w_{A} aus den einzelnen Wassergehalten von Inert, Biogen und Fossil. Wertebereiche für die Wassergehalte dieser drei Gruppen lassen sich aus Sortieranalysen und Literaturangaben ableiten. ${w}_{I} ⋅ {w}_{I} + {m}_{B} ⋅ {w}_{B} + {m}_{F} ⋅ {w}_{F} = {w}_{A}$

Da bei der Verbrennung von Abfällen durch die Oxidation von Wasserstoff zusätzliches Wasser gebildet wird, gilt es diese Wassermenge in der Bilanz ebenso zu berücksichtigen. Die durch Wasserstoffoxidation gebildete Wassermenge errechnet sich folgendermaßen (Elementargehalte sind auf wasserfreie aber aschehältige Substanz bezogen): $\frac{18}{2} ⋅ \left({m}_{B}⋅\left(1-{w}_{B}\right)⋅{c}_{{H}_{B}}+{m}_{F}⋅\left(1-{w}_{F}\right)⋅{c}_{{H}_{F}}\right) = {w}_{H}$

Sofern sich die Angaben über die Elementargehalte auf die wasser- und aschefreie Substanz beziehen, ist die Gleichung zur Berechnung der Wasserbildung folgendermaßen zu ergänzen: $\frac{18}{2} ⋅ \left({m}_{B}⋅\left(1-{w}_{B}\right)⋅\left(1-{a}_{B}\right)⋅{c}_{{H}_{B}}+{m}_{F}⋅\left(1-{w}_{F}\right)⋅\left(1-{a}_{F}\right)⋅{c}_{{H}_{F}}\right) = {w}_{H}$

Die Summe beider rechnerisch ermittelter Wassermengen w_{A} und w_{H} muss mit dem "scheinbaren" Wassergehalt des Abfalls w_{S} (beinhaltet den "eigentlichen" Wassergehalt und jenes Wasser, das durch Wasserstoffoxidation entsteht), der sich aus den Betriebsdaten der Verbrennungsanlage (Wasserdampfgehalt im Reingas, Reingasmenge, Wasserbilanz des Wäschers) berechnen lässt, übereinstimmen. ${w}_{A} + {w}_{H} = {w}_{S}$

Im Rahmen angestellter Berechnungen und Überlegungen zeigt sich, dass die Einbeziehung der Wasserbilanzgleichung für das zu bestimmende Ergebnis (biogener bzw. fossiler Anteil an Kohlenstoff bzw. Heizwert) von ungeordneter Bedeutung ist. Grund dafür ist einerseits die hohe Unsicherheit von Wasserbilanzen in Verbrennungsanlagen und andererseits der ähnliche Informationsgehalt im Vergleich zu den anderen Gleichungen. In einer Variante der Erfindung kann jedoch die Wasserbilanz sehr wohl als zusätzliche Bilanzgleichung in die Ermittlung der einzelnen Anteile aufgenommen werden.

### Theoretische Güter-, Stoff-, und Energiebilanzen für die Variante mit 4 Stoffgruppen

In Folge der Unterteilung in 4 Stoffgruppen (inert, biogen, fossil und Wasser) sind in den biogenen und fossilen Massenanteilen dieser Variante kein Wasser und keine Asche und in den inerten Massenanteilen kein Wasser enthalten.

### 1) "Massenbilanz":

${m}_{I} + {m}_{B} + {m}_{F} + {m}_{W} = 1$

### 2) Aschen"-Bilanz":

${m}_{I} = {a}_{A} ⋅ \left(1-{w}_{A}\right)$

### 3) "Kohlenstoffbilanz":

${m}_{B} ⋅ {c}_{{C}_{B}} + {m}_{F} ⋅ {c}_{{C}_{F}} = {c}_{{C}_{A}}$

### 4) "Energie-Bilanz":

$\begin{matrix}{m}_{B} ⋅ \left(\begin{matrix}34 , 834 ⋅ {c}_{{C}_{B}} + 93 , 868 ⋅ {c}_{{H}_{B}} - 10 , 802 ⋅ {c}_{{O}_{B}} + \\ + 6 , 28 ⋅ {c}_{{N}_{B}} + 10 , 467 ⋅ {c}_{{S}_{B}}\end{matrix}\right) + \\ + {m}_{F} ⋅ \left(\begin{matrix}34 , 834 ⋅ {c}_{{C}_{F}} + 93 , 868 ⋅ {c}_{{H}_{F}} - 10 , 802 ⋅ {c}_{{O}_{F}} + \\ + 6 , 28 ⋅ {c}_{{N}_{F}} + 10 , 467 ⋅ {c}_{{S}_{F}}\end{matrix}\right) + {m}_{W} ⋅ \left(-2,449\right) = {H}_{u , A , B}\end{matrix}$ ${H}_{u , A} = 0 , 9806 ⋅ {H}_{u , A , B} - 0 , 495$

### 5) "Sauerstoffumsatzbilanz"

$\begin{matrix}{m}_{B} ⋅ \left(\frac{{c}_{{C}_{B}}}{12}+\frac{{c}_{{H}_{B}}}{4}-\frac{{c}_{{O}_{B}}}{32}+\frac{{c}_{{N}_{B}}}{14}+\frac{{c}_{{S}_{B}}}{32}\right) ⋅ 1000 + {m}_{F} ⋅ \left(\frac{{c}_{{C}_{F}}}{12}+\frac{{c}_{{H}_{F}}}{4}-\frac{{c}_{{O}_{F}}}{32}+\frac{{c}_{{N}_{F}}}{14}+\frac{{c}_{{S}_{F}}}{32}\right) ⋅ 1000 = {O}_{2} ⁢ V\end{matrix}$

### 6) "Sauerstoffverbrauchs- und Kohlendioxidproduktions"-Bilanz

$\begin{matrix}{m}_{B} ⋅ \left(\frac{{c}_{{H}_{B}}}{4}-\frac{{c}_{{O}_{B}}}{32}+\frac{{c}_{{N}_{B}}}{14}+\frac{{c}_{{S}_{B}}}{32}\right) ⋅ 1000 + {m}_{F} ⋅ \left(\frac{{c}_{{H}_{F}}}{4}-\frac{{c}_{{O}_{F}}}{32}+\frac{{c}_{{N}_{F}}}{14}+\frac{{c}_{{S}_{F}}}{32}\right) ⋅ 1000 = {Δ}_{O ⁢ 2 + CO ⁢ 2 , g}\end{matrix}$

### 7) "Wasserbilanz":

${m}_{B} ⋅ \left(\frac{18}{2}⋅{c}_{{H}_{B}}\right) + {m}_{F} ⋅ \left(\frac{18}{2}⋅{c}_{{H}_{F}}\right) + {m}_{W} = {w}_{S}$

### Sonstiges:

In beiden Berechnungsvarianten (mit 3 oder 4 Stoffgruppen) sind zusätzlich zu den angeführten Bilanzgleichungen noch folgende Bedingungen zu erfüllen:

Die Summe der Elementargehalte (C-, H-, O-, N-, S-Gehalt bezogen auf wasser- und aschefreie Substanz) der biogenen und fossilen Stoffgruppen muss 1 bzw. kleiner gleich 1 sein.

Für biogene Materialien kann angenommen werden, dass die Summe aus C, H, O, N und S 1 ergibt, während für fossile Materialien (je nach PVC-Anteil und damit Chloridgehalt) ein etwas geringerer Wert als 1 angenommen werden kann. ${c}_{{C}_{B}} + {c}_{{H}_{B}} + {c}_{{O}_{B}} + {c}_{{N}_{B}} + {c}_{{S}_{B}} \approx < 1$ ${c}_{{C}_{F}} + {c}_{{H}_{F}} + {c}_{{O}_{F}} + {c}_{{N}_{F}} + {c}_{{S}_{F}} \approx 0 , 98 \pm 0 , 01$

### "Messbare" Güter-, Stoff und Energiebilanzen der Verbrennungsanlage

### 1) Massenermittlung:

Die Masse des verbrannten Mülls wird bei der Aufgabe des Abfalls mit der direkt im Greifer des Müllkrans integrierten Kranwaage mechanisch - elektrisch gewogen. Die Masse des behandelten Mülls wird benötigt, um alle weiteren Betriebsdaten der Verbrennungsanlage auf die Abfallmenge beziehen zu können.

### 2) Aschen-Bilanz:

Die Summe der Massen der festen Rückstände (Schlacke, Schrott, Kesselasche und Elektrofilterasche) aus der Verbrennungsanlage bezogen auf die Masse des Abfallinputs ergibt den Aschegehalt.

Die Massen der einzelnen Rückstände werden diskontinuierlich durch Wägung erfasst. Da die Rückstände zwar trocken anfallen, jedoch vor ihrer Wägung verschiedene Behandlungsschritte durchlaufen, sind zur Bestimmung des Aschegehaltes zusätzlich zur Wägung der Massen, Angaben über den Wassergehalt der Rückstände (verursacht durch nasse Schlacken- und Aschenbehandlung) notwendig. Rauchgasreinigungsmittel (NaOH oder CaO) in den Rückständen (Aschen) sind mengenmäßig für die Bilanz unbedeutend. ${a}_{A} ⋅ \left(1-{w}_{A}\right) = \frac{{M}_{Schlacke} + {M}_{Schrott} + {M}_{Kesselasche} + {M}_{EF - Asche}}{{M}_{Abfall}}$

### 3) Kohlenstoffbilanz:

Aus der Kohlendioxidfracht im Reingas lässt sich der Kohlenstoffgehalt des verbrannten Abfalls berechnen. Die C-Fracht im Reingas muss jedoch um jene Kohlenstoffmenge verringert werden, die sich aus der Feuerung von eingesetzten Zusatzbrennstoffen (Ergas, Erdöl) ergibt.

Im Routinebetrieb werden sowohl die Rauchgasmenge als auch die Kohlendioxidkonzentration online über das Leitsystem erfasst.

Mit dieser Information ist es möglich, den Kohlenstoffgehalt des Abfalls laufend (ohne zusätzlichen Messaufwand) zu ermitteln. ${c}_{{C}_{A}} = \frac{\left({V}_{R}⋅{c}_{{CO}_{2 , R}}⋅\frac{12}{22 , 4}-{M}_{Erdgas}⋅{c}_{C , Erdgas}\right)}{{M}_{Abfall} ⋅ {T}_{C , R}}$

Zur Umrechnung der volumetrischen CO2-Fracht (Produkt aus Reingasvolumenstrom und CO2-Konzentration im Reingas) auf eine Massenfracht an Kohlenstoff ist die volumetrische C02-Fracht durch das Molvolumen (22,4 Liter) zu dividieren und mit dem Molgewicht des Kohlenstoffs (12 g) zu multiplizieren.

Zur exakteren Bestimmung des organischen Kohlenstoffgehaltes, der tatsächlich zu CO₂ (umgesetzt) verbrannt wurde, empfiehlt es sich, anstelle der oberen Gleichung folgende Gleichung heranzuziehen (Konzentrationsangaben der Verbrennungsluft und des Reingases sind hier in Prozentzahlen einzusetzen): ${c}_{{C}_{A}} = \frac{\left({V}_{R}⋅\left({c}_{{CO}_{2 , R}}-\frac{100 - {c}_{{O}_{2 , R}} - {c}_{{CO}_{2 , R}}}{100 - {c}_{{O}_{2 , L}} - {c}_{{CO}_{2 , L}}}⋅{c}_{{CO}_{2 , L}}\right)⋅\frac{1}{100}⁢\frac{12}{22 , 4}-{M}_{Erdgas}⋅{c}_{C , Erdgas}\right)}{{M}_{Abfall}}$

### 4) Energie-Bilanz:

Der Heizwert des Abfalls wird aus den Betriebsdaten, im Speziellen aus der produzierten Dampfmenge, der Netto-Enthalpie des Wassers im Kessel und dem Wirkungsgrad der Anlage berechnet. Die Bilanzgrenze des angegebenen Wirkungsgrades muss mit jener Grenze, die zur Bestimmung der Nettoenthalpie des Wassers im Kessel herangezogen wird, übereinstimmen. Die der Anlage über Zusatzbrennstoffe (Erdgas, Erdöl) zugeführte Energie ist ebenfalls zu berücksichtigen. ${H}_{u , A} = \frac{WD ⋅ \left({h}_{T}^{ʺ}-{h}_{K}^{ʹ}\right)}{{M}_{Abfall} ⋅ η} - \frac{{H}_{u , Erdgas} ⋅ {M}_{Erdgas}}{{M}_{Abfall}}$

### 5) Sauerstoffumsatzbilanz

Der O₂-Verbrauch bei Müllverbrennung lässt sich aus den Betriebsdaten folgendermaßen berechnen (Konzentrationsangaben der Verbrennungsluft und des Reingases sind hier in Prozentzahlen einzusetzen): ${O}_{2} , V = \frac{{V}_{R} ⋅ \left({c}_{O ⁢ 2 , L}⋅\left(-\frac{100 - {c}_{O ⁢ 2 , R} - {c}_{CO ⁢ 2 , R}}{100 - {c}_{O ⁢ 2 , L} - {c}_{CO ⁢ 2 , L}}\right)-{c}_{O ⁢ 2 , R}\right) ⋅ \frac{1}{100 ⋅ 0 , 0224} - \left(\frac{{M}_{H , Erdgas}}{4}+\frac{{c}_{C , Erdgas}}{12}\right) ⋅ {M}_{Erdgas} ⋅ 1000}{{M}_{Abfall}}$

### 6) Sauerstoffverbrauchs- und Kohlendioxidproduktions-Bilanz

Die Differenz aus O₂-Verbrauch und CO₂-Produktion bei Müllverbrennung lässt sich aus den Betriebsdaten folgendermaßen berechnen: ${Δ}_{O ⁢ 2 + CO ⁢ 2 , g} = \frac{\left({c}_{O ⁢ 2 , L}+{c}_{CO ⁢ 2 , L}\right) - \left({c}_{O ⁢ 2 , R}+{c}_{CO ⁢ 2 , R}\right)}{100 ⋅ {m}_{V}} ⋅ \frac{{V}_{R} ⋅ \left(\frac{79 + \left({c}_{O ⁢ 2 , L}+{c}_{CO ⁢ 2 , L}\right)}{79 + \left({c}_{O ⁢ 2 , R}+{c}_{CO ⁢ 2 , R}\right)}\right)}{{M}_{Abfall}}$

Zur exakteren Bestimmung der Sauerstoffverbrauchs- und Kohlendioxidproduktions-Bilanz unter Berücksichtigung der Zusatzbrennstoffe, empfiehlt es sich, folgende Gleichung heranzuziehen (Konzentrationsangaben der Verbrennungsluft und des Reingases sind hier in Prozentzahlen einzusetzen): ${Δ}_{O ⁢ 2 + CO ⁢ 2 , R} = \frac{{V}_{R} ⋅ \left(\left({c}_{O ⁢ 2 , L}+{c}_{CO ⁢ 2 , L}\right)⋅\left(-\frac{100 - {c}_{O ⁢ 2 , R} - {c}_{CO ⁢ 2 , R}}{100 - {c}_{O ⁢ 2 , L} - {c}_{CO ⁢ 2 , L}}\right)-\left({c}_{O ⁢ 2 , R}+{c}_{CO ⁢ 2 , R}\right)\right) ⋅ \frac{1}{100 ⋅ 0 , 0224} - \frac{{M}_{H , Erdgas}}{4} ⋅ {M}_{Erdgas} ⋅ 1000}{{M}_{Abfall}}$

### 7) Wasserbilanz:

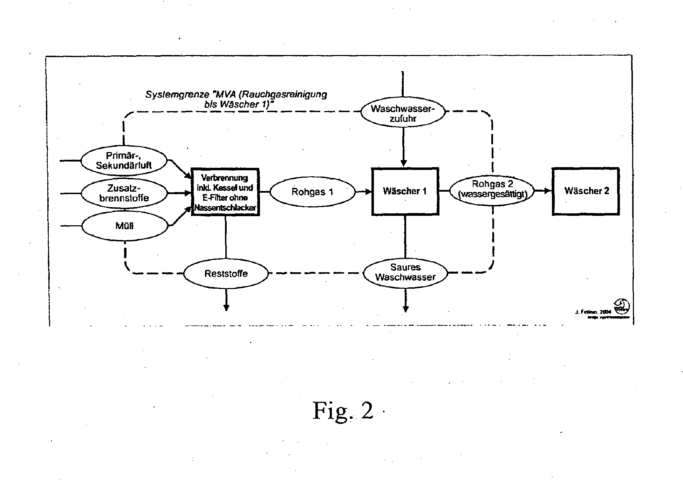

Der mittlere Wassergehalt des Abfalls lässt sich anhand einer Wasserbilanzierung des Systems Verbrennungsanlage (Verbrennung inkl. Kessel, E-Filter und nasser Rauchgasreinigung) ermitteln. Die einzelnen Güterflüsse dieses Systems (siehe Fig. 2) werden mit Ausnahme des Waschwasserinputs im Routinebetrieb laufend erfasst.

### - Wasserinput über Primärluft und Sekundärluft:

Die Menge an Primär- und Sekundärluft wird i.d.R. als Volumenstrom bezogen auf Normalbedingungen mittels einer Venturidüse online gemessen und über das Leitsystem der Anlage erfasst. Bei Kenntnis der Lufttemperatur und der relativen Luftfeuchte (wird üblicherweise ebenfalls gemessen) lässt sich der Wasserinput über die Primär- und Sekundärluft folgendermaßen errechnen: $e = RF ⋅ 0 , 6108 ⋅ {e}^{\frac{\left(17,27⋅T\right)}{{L}_{Left} + 237 , 3}} {a}_{PL - SL} = \frac{{m}_{mol , H ⁢ 20}}{R} ⋅ \frac{e}{273 , 15 + {T}_{Luft}}$ ${a}_{N , PL - SL} = \frac{{T}_{Luft} + 273 , 15}{273 , 15} ⋅ \frac{{p}_{N}}{{p}_{Luft}} {m}_{H ⁢ 2 ⁢ O , PL - SL} = {V}_{PL - SL} ⋅ {a}_{N , PL - SL}$

### - Wasserinput über den Abfall:

Der Wassereintrag über den Abfall setzt sich zusammen aus dem "eigentlichen" Wassergehalt des Mülls w_{A} und dem "chemischen" Wasserinput w_{H}, der aus der anschließenden Oxidation des Wasserstoffs im Müll herrührt. Die Unbekannte, die es im Rahmen der Wasserbilanzierung zu berechnen gilt, ist die Summe w_{S} der beiden Wassereinträge.

### - Wasserinput über Zusatzbrennstoffe:

Der scheinbare Wassereintrag über Zusatzbrennstoffe (Erdöl, Erdgas) lässt sich aus der eingesetzten Brennstoffmenge und deren Gehalt an Wasserstoff folgendermaßen abschätzen. ${m}_{H ⁢ 20 , Erdgas} = {M}_{Erdgas} ⋅ {c}_{H , Erdgas} ⋅ \frac{18}{2}$

### - Wasserinput und -output in den - aus dem Wäscher (Waschwasserzufuhr);

Im Fall der nassen Rauchgaswäsche ist die dem Rauchgaswäscher zugeführte und abgezogene Wassermenge zu messen.

### - Wasseroutput über die Reststoffe:

Die Reststoffe (Schlacke, Schrott, Kessel-Asche, E-Filterasche) der Verbrennung fallen i.d.R. trocken an (der Nassentschlacker liegt außerhalb des Systems). Somit wird über diese Güter kein Wasser aus dem System ausgeschleust.

### - Wasseroutput über das Rohgas 2 (nach dem Wäscher):

Wie aus Fig. 2 ersichtlich ist, wird im Fall einer nassen Rauchgasreinigung das Rohgas im Wäscher 1 vollständig mit Wasserdampf gesättigt. Bei bekannter Temperatur (wird i.d.R. online gemessen) und bekanntem Druck im Wäscher lässt sich der absolute Wassergehalt im Rohgas 2 berechnen (siehe nachfolgende Gleichungen). Für die Ermittlung der Menge des Rohgases 2 kann angenommen werden, dass diese der Reingasmenge entspricht, wobei die Reingasmenge als Volumenstrom bezogen auf Normalbedingungen online über das Leitsystem erfasst wird. ${p}_{a} = 0 , 6108 ⋅ {e}^{\frac{\left(17,27⋅T\right)}{{T}_{W ⁢ {a}^{¨} ⁢ scher} + 237 , 3}} {a}_{W ⁢ {a}^{¨} ⁢ scher} = \frac{{m}_{mol , H ⁢ 20}}{R} ⋅ \frac{{p}_{a}}{273 , 15 + {T}_{Wascher}}$ ${a}_{N , Rohgas ⁢ 2} = \frac{{T}_{W ⁢ {a}^{¨} ⁢ scher} + 273 , 15}{273 , 15} ⋅ \frac{{p}_{N}}{{p}_{W ⁢ {a}^{¨} ⁢ scher}} {m}_{H ⁢ 2 ⁢ O , Rohgas} = {V}_{R} ⋅ {a}_{N , Rohgas ⁢ 2}$

### Mathematische Lösung der Bilanzgleichungen

Ein Zusammenführen der vorgestellten Bilanzgleichungen führt zu einem linearen Gleichungssystem von sieben Gleichungen mit drei Unbekannten m_{I}, m_{B} und m_{F} (Massenanteil Inert, Biogen und Fossil). Es handelt sich somit um ein überbestimmtes System, dessen Lösung über eine Ausgleichrechnung ermittelt werden muss. Die Koeffizienten (c_{CB}, c_{CF}, ...) der Unbekannten und die Anlagenmesswerte sind durch Mittelwerte (wahrscheinlichste Werte) und Unsicherheitsbereiche gegeben.

Um die notwendige nichtlineare Ausgleichsrechnung zu umgehen und zu einer Näherungslösung zu gelangen, kann folgende Vorgangsweise gewählt werden: Die Koeffizienten der Unbekannten werden gemäß ihrer Unsicherheiten mittels Monte Carlo Simulation variiert. Für jede dabei entstehende Kombination von Koeffizienten wird eine lineare Ausgleichsrechung durchgeführt. Da in jeder Gleichung genau eine Beobachtungsgröße (Anlagenmesswert) als lineare Funktion der Unbekannten vorkommt, kann das Gauß-Markov-Modell angewandt werden: $l + v = Ax$ ${Σ}_{u} = {σ}_{0}^{2} ⁢ {Q}_{u}$
mit
*l* Vektor der Beobachtungen
*v* Vektor der Verbesserungen
*A* Koeffizientenmatrix der Unbekannten
*x* Vektor der Unbekannten,
Σ*_{II}* Kovarianzmatrix der Beobachtungen
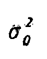 Varianz der Gewichtseinheit
*Q_{II}* Kofaktormatrix der Beobachtungen

Daraus ergeben sich die Schätzwerte für die Unbekannten *x̂* inklusive ihrer Kofaktormatrix *Q*ₓₓ : $\hat{x} = {\left({A}^{T}⁢{{Q}_{u}}^{- 1}⁢A\right)}^{- 1} ⁢ {A}^{T} ⁢ {{Q}_{u}}^{- 1} ⁢ l$ ${Q}_{xx} = {\left({A}^{T}⁢{{Q}_{u}}^{- 1}⁢A\right)}^{- 1}$

Die ausgeglichenen Beobachtungen *î* inklusive ihrer Kofaktormatrix *Qᵢᵢ* berechnen sich aus: $\hat{l} = A ⁢ \hat{x}$ ${Q}_{\hat{i} ⁢ \hat{i}} = A ⁢ {Q}_{xx} ⁢ {A}^{T}$

Entsprechend der Anzahl der Wiederholungen der Monte Carlo Simulation erhält man dadurch sowohl für jede Unbekannte *x* als auch für jede Beobachtung *l* die gleiche Anzahl möglicher Werte. Diese Werte werden statistisch ausgewertet und durch Parameter (Mittelwert, Standardabweichung) beschrieben.

Genauere Ergebnisse erhält man durch die Anwendung einer nichtlinearen Ausgleichsrechnung (Narasimhan et al, 2000), bei der die Unsicherheit der Koeffizienten direkt (ohne Umweg über eine Monte Carlo Simulation) berücksichtigt werden können.

### Beispiel:

### Verwendete Daten

Zur Bestimmung der Koeffizienten (Wassergehalt, Aschegehalt, Kohlenstoffgehalt, Wasserstoffgehalt, Sauerstoffgehalt, Schwefelgehalt, Stickstoffgehalt) der Unbekannten (Massenanteile m_{I}, m_{B} und m_{F} bzw. m_{w}) in den "theoretischen" Bilanzgleichungen können Ergebnisse von Sortieranalysen und Analysen hinsichtlich der Elementarzusammensetzung einzelner Abfallfraktionen herangezogen werden. In einem exemplarisch dargelegten Beispiel stützen sich die errechneten Koeffizienten (für die Variante, die vorsieht, dass der Abfall in drei Stoffgruppen unterteilt wird) auf Sortieranalysen von TBHauer (2000) durchgeführt am Input einer Verbrennungsanlage und Untersuchungen zur brennstoff-technischen Charakterisierung von Haushaltsabfällen (Kost, 2001).

Alternativ dazu können für die Variante, die vorsieht, dass der Abfall in vier Stoffgruppen unterteilt wird, die benötigten Koeffizienten der fossilen Stoffgruppe (Kohlenstoffgehalt, Wasserstoffgehalt, Sauerstoffgehalt, Schwefelgehalt, Stickstoffgehalt) auch über Konsumzahlen, Anwendungsbereiche, mittlere Aufenthaltsdauern und Entsorgungswege der Polymere berechnet werden.

Für den Fall, dass in der zu untersuchenden Verbrennungsanlage überwiegend Restmüll verbrannt wird, können die im Folgenden angeführten Werte als allgemein gültige Standardwerte herangezogen werden. Werden in der betrachteten Anlage andere Abfallarten in überwiegendem Maße behandelt, (beispielsweise Einzelfraktionen aus der mechanisch biologischen Abfallbehandlung, oder verschiedene Sortierreste), so ist zur Bestimmung der Ausgangsgrößen (die Koeffizienten: Wassergehalt, Aschegehalt, Kohlenstoffgehalt, Wasserstoffgehalt, Sauerstoffgehalt, Schwefelgehalt, Stickstoffgehalt) eine einmalige Sortieranalyse empfehlenswert. Relevante Änderungen in den Ausgangsgrößen sind jedoch nur für die Parameter Wassergehalt und Aschegehalt zu erwarten.

Bei Anwendung der Variante, die vorsieht, dass der Abfall in vier Stoffgruppen unterteilt wird, können die benötigten Ausgangsgrößen (Kohlenstoffgehalt, Wasserstoffgehalt, Sauerstoffgehalt, Schwefelgehalt, Stickstoffgehalt der biogenen und fossilen Materialien) universell für alle gemischten Abfälle eingesetzt werden.

Im Folgenden werden 2 Beispiele für die Bestimmung der mittleren Stoffeigenschaften gegeben:

### Bestimmung der mittleren Stoffeigenschaften

### 1) Kombination von Sortieranalysen und Informationen über die Elementar-zusammensetzung der einzelnen Sortierfraktionen

### Sortieranalysen von TBHauer (2000)

Im Zeitraum vom 14. bis 25. Februar 2000 wurden von TBHauer am Werksgelände der zu untersuchenden Müllverbrennungsanlage Sortieranalysen des angelieferten Abfalls durchgeführt. Die Analysen erfolgten dabei getrennt für Restmüll sowie für Gewerbe- und Spemnüll. Die fraktionelle Zusammensetzung wurde mittels manueller Sortierung bestimmt. Die Ergebnisse der Analysen sind in Tabelle 2 und Tabelle 3 zusammengefasst. Der Wassergehalt wurde durch Trocknen von Proben aus den einzelnen Fraktionen bestimmt.

**Tabelle 2 Abfallzusammensetzung (Fraktionen) des angelieferten Restmülls (TBHauer, 2000)**

| **Fraktion** | **Restmüll [m% FS]** | | **Wassergehalt [m% FS]** | | **Restmüll [m% TS]** | |
|---|---|---|---|---|---|---|
| | **Mittelw.** | **Stdabw.** | **Mittelw.** | **Stdabw.^{a}** | **Mittelw.** | **Stdabw.** |
| Papier | 13,3 | 2,3 | 20 | 10 | 10,6 | 2,3 |
| Glas | 4,2 | 1,0 | 2 | 1 | 4,1 | 1,0 |
| Metalle | 5,2 | 1,1 | 7 | 2 | 4,8 | 1,0 |
| Holz | 3,2 | 1,3 | 17 | 4 | 2,7 | 1,1 |
| Hygienewaren | 10,7 | 2,2 | 45 | 6 | 5,9 | 1,4 |
| Biogenes | 14,7 | 2,8 | 60 | 6 | 5,9 | 1,4 |
| Rückstände aus Kompostierung | 3,7 | | 75 | 5 | 0,9 | 0,2 |
| Textilien | 10,3 | 2,0 | 20 | 14 | 8,2 | 2,2 |
| Kunststoffe | 17,5 | 2,4 | 10 | 7 | 15,8 | 2,5 |
| Inertes | 11,4 | 3,0 | 8 | 2 | 10,5 | 2,8 |
| Materialverbunde | 5,8 | 1,5 | 15 | 6 | 4,9 | 1,3 |
| *Summe* | *100,0* | | | | *74,3* | *5,7* |

| | | | | | | |
|---|---|---|---|---|---|---|
| *^{a} Die Standardabweichung des Wassergehaltes wurde basierend auf einer Zusammenstellung von Literaturdaten (Kost, 2001) angenommen* | | | | | | |

**Tabelle 3 Abfallzusammensetzung (Fraktionen) des angelieferten Gewerbe- und Sperrmülls (TBHauer, 2000)**

| **Fraktion** | **Gewerbe- und Sperrmüll [m% FS]** | | **Wassergehalt [m% FS]** | | **Gewerbe-und Sperrmüll [m% TS]** | |
|---|---|---|---|---|---|---|
| | **Mittelw.** | **Stdabw.^{a}** | **Mittelw.** | **Stdabw.^{b}** | **Mittelw.** | **Stdabw.** |
| Papier | 12,0 | 1,8 | 20 | 10 | 9,6 | 1,9 |
| Glas | 0,2 | 0,0 | 2 | 1 | 0,2 | 0,0 |
| Metalle | 3,9 | 0,6 | 7 | 2 | 3,6 | 0,5 |
| Holz | 28,0 | 4,2 | 17 | 4 | 23,2 | 3,7 |
| Hygienewaren | 0,1 | 0,0 | 45 | 6 | 0,1 | 0,0 |
| Biogenes | 3,1 | 0,5 | 60 | 6 | 1,2 | 0,3 |
| Rückstände aus Kompostierung | | 0,0 | 75 | 5 | 0,0 | 0,0 |
| Textilien | 3,9 | 0,6 | 20 | 14 | 3,1 | 0,7 |
| Kunststoffe | 31,0 | 4,7 | 10 | 7 | 27,9 | 4,7 |
| Inertes | 9,3 | 1,4 | 8 | 2 | 8,6 | 1,3 |
| Materialverbunde | 8,5 | 1,3 | 15 | 6 | 7,2 | 1,2 |
| ***Summe*** | ***100,0*** | | | | ***84,8*** | ***6,6*** |

| | | | | | | |
|---|---|---|---|---|---|---|
| *^{a} Die Standardabweichung der Zusammensetzung wurde mit 15 % des Mittelwertes angenommen* *^{b} Die Standardabweichung des Wassergehaltes wurde basierend auf einer Zusammenstellung von Literaturdaten (Kost, 2001) angenommen* | | | | | | |

Um für die einzelnen "Stoffgruppen" Inert, Biogen und Fossil mittlere (wahrscheinlichste) Zusammensetzungen (Wasser-, Asche, Kohlenstoffgehalt) berechnen zu können, müssen die einzelnen Sortierfraktionen den drei "Gruppen" zugeordnet werden. Bei jenen Fraktionen (Hygienewaren, Textilien, Materialverbunde) die sowohl biogene als auch fossile Energieträger enthalten, erfolgte die Aufteilung gemäß Literaturdaten bzw. wurde nach Angaben zur Sortieranalyse (TBHauer, 2000) berechnet, siehe Tabelle 4.

**Tabelle 4 Zuordnung zu den "Stoffgruppen"**

| | **Massenanteile [m%]** | | |
|---|---|---|---|
| | **Inert** | **Biogen** | **Fossil** |
| Papier | - | 100 | - |
| Glas | 100 | - | - |
| Metalle | 100 | - | - |
| Holz | - | 100 | - |
| Hygienewaren | - | 43^{a} | 57^{a} |
| Biogenes | - | 100 | |
| Rückstände aus Kompostierung | - | 100 | |
| Textilien | - | 64^{b} | 36^{b} |
| Kunststoffe | - | - | 100 |
| Inertes | 100 | - | - |
| Materialverbunde Hausmüll | - | 24,6^{c} | 75,4^{c} |
| Materialverbunde Gewerbemüll | | 41,1^{c} | 58,9^{c} |

| | | | |
|---|---|---|---|
| *" Aufteilung gemäß Windelzusammensetzung: 43 % zehstoffflocken, 27 % Superabsorber, 10 % Polypropylen, 13 % Polyethylen, 7 % Klebstoffe, Klebebänder (EDANA, 2001)* *^{b} Aufteilung gemäß Weltproduktionsdaten der Textilindustrie 1990 (Echte, 1993)* *^{c} Aufteilung gemäß Angaben zur Sortieranalyse (TBHauer, 2000)* | | | |

### Elementarzusammensetzung von Sortierfraktionen (Kost, 2001)

Im Rahmen einer Studie zur brennstofftechnischen Charakterisierung von Haushaltsabfällen wurde von Kost (2001) die stoffliche Zusammensetzung von Sortierfraktionen bestimmt. Dabei wurden jeweils Proben von ca. 20 kg in einer kleintechnischen Verbrennungsanlage verbrannt. Aus den gemessenen Emissionen der Anlage wurde die Elementarzusammensetzung (C, H, O, N, S, Cl, Aschegehalt und Wassergehalt) der Abfallproben rückgerechnet. Die Ergebnisse dieser Untersuchungen sind in der folgenden Tabelle 5 zusammengefasst.

**Tabelle 5 Elementarzusammensetzung von Abfallfraktionen (näch Kost, 2001)**

| **Fraktion** | **Gruppe** | **Aschegehalt** | | **Kohlenstoffgehalt** | | **Wasserstoffgehalt** | | **Schwefelgehalt** | | **Stickstoffgehalt** | | **Sauerstoffgehalt** | |
|---|---|---|---|---|---|---|---|---|---|---|---|---|---|
| | | **[m% TS]** | | **[kg/kg aschefrei]** | | | | | | | | | |
| | | Mittelw. | Stabw. | Mittelw. | Stabw. | Mittelw. | Stabw. | Mittelw. | Stabw. | Mittelw. | Stabw. | Mittelw. | Stabw. |
| Papier | biogen | 18 | 6 | 0,460 | 0,010 | 0,066 | 0,0015 | 0,003 | 0,0005 | 0,006 | 0,0005 | 0,460 | 0,015 |
| Glas | inert | 100 | - | - | - | - | - | - | - | - | - | - | - |
| Metalle | inert | 100 | - | - | - | - | - | - | - | - | - | - | - |
| Holz | biogen | 5 | 4,2 | 0,480 | 0,010 | 0,064 | 0,001 | 0,004 | 0,002 | 0,012 | 0,005 | 0,440 | 0,010 |
| Hygienewaren_biogen^{a} | biogen | 10 | 3 | 0,440 | 0,012 | 0,062 | 0,001 | 0,002 | 0,0005 | 0,008 | 0,002 | 0,470 | 0,015 |
| Hygienewaren_fossil^{a} | fossil | 10 | 3 | 0,625 | 0,013 | 0,096 | 0,0035 | 0,002 | 0,0005 | 0,008 | 0,002 | 0,250 | 0,026 |
| Biogenes | biogen | 21 | 15 | 0,500 | 0,026 | 0,075 | 0,005 | 0,003 | 0,0005 | 0,027 | 0,0035 | 0,390 | 0,031 |
| Rückstände aus Kompostierung^{b} | biogen | 21 | 15 | 0,500 | 0,026 | 0,075 | 0,005 | 0,003 | 0,0005 | 0,027 | 0,0035 | 0,390 | 0,031 |
| Textilien_biogen | biogen | 9 | 6 | 0,445 | 0,014 | 0,062 | 0,002 | 0,004 | 0,0015 | 0,012 | 0,003 | 0,474 | 0,012 |
| Textilien_fossil | fossil | 9 | 6 | 0,645 | 0,044 | 0,064 | 0,003 | 0,004 | 0,0015 | 0,063 | 0,010 | 0,227 | 0,024 |
| Kunststoffe | fossil | 18 | 8 | 0,750 | 0,046 | 0,111 | 0,011 | 0,003 | 0,0015 | 0,008 | 0,001 | 0,110 | 0,056 |
| Inertes | inert | 100 | - | - | - | - | - | - | - | - | - | - | - |
| Materialverbunde_biogen^{c} | biogen | 39 | 20 | 0,460 | 0,010 | 0,066 | 0,0015 | 0,003 | 0,0005 | 0,006 | 0,0005 | 0,460 | 0,015 |
| Materialverbunde_fossil^{c} | fossil | 39 | 20 | 0,750 | 0,046 | 0,111 | 0,011 | 0,003 | 0,0015 | 0,008 | 0,001 | 0,110 | 0,056 |

| | | | | | | | | | | | | | |
|---|---|---|---|---|---|---|---|---|---|---|---|---|---|
| *^{a} Zusammensetzung laut eigenen Berechnungen* *^{b} Zusammensetzung analog zu Biogenem angenommnen* ^{c} *Annahme: der biogene Anteil an Materialverbunden entspricht in der Elementar-Zusammensetzung jener von Papier und der fossile Anteil entspricht jener der Kunststofffraktion* | | | | | | | | | | | | | |

### Zusammensetzung der Gruppen Inert, Biogen und Fossil

Aus den Ergebnissen der Sortieranalysen (Tabelle 2), der Zuordnung zu den Stoffgruppen (Tabelle 4) und der Elementarzusammensetzung der einzelnen Sortierfraktionen (Tabelle 5) lassen sich für die drei Gruppen (Inert, Biogen und Fossil) mittlere Zusammensetzungen errechnen. Unter Berücksichtigung der Unsicherheiten aller Eingangsdaten über die Fehlerfortpflanzungsrechnung nach Gauß, können zusätzlich zu den Mittelwerten deren Standardabweichungen ausgewiesen werden.

Da sich aufgrund der unterschiedlichen fraktionellen Zusammensetzung von Restmüll und Gewerbemüll, auch unterschiedliche Elementarzusammensetzungen der drei Stoffgruppen Inert, Biogen und Fossil ergeben, werden diese in Tabelle 6 für beide Abfällarten getrennt angegeben.

Für die anschließenden Ausgleichsrechnungen, empfiehlt es sich nicht mehr zwischen Hausmüll und Gewerbemüll zu unterschieden. Es ist daher erforderlich die Daten aus Tabelle 6 zu mittleren Gehalten an Wasser, Asche, C, H, S, N und O zusammenzuführen (siehe Tabelle 7). Der Anteil des Restmülls kann i.d.R. aus den Betriebsdaten über das Geschäftsjahr ermittelt werden (im vorliegenden Beispiel beträgt dieser rund 75 %). Die Vereinfachung (Verwendung eines mittleren jährlichen Hausmüll- bzw. Gewerbemüllanteils) ist aufgrund der sehr ähnlichen Elementarzusammensetzung von Haus- und Gewerbemüll zulässig. Darüber hinaus sind die verwendeten Unsicherheitsbereiche deutlich größer als die einzelnen Unterschiede in der Zusammensetzung.

**Tabelle 6 Elementarzusammensetzung der "Stofffgruppen " für Restmüll sowie Gewerbemüll und Sperrmüll (aus Sortieranalysen bestimmt)**

| | **Gruppe** | **Wassergehalt** | | **Aschegehalt** | | **Kohlenstoffgehalt** | | **Wasserstoffgehalt** | | **Schwefelgehalt** | | **Stickstoff gehalt** | | **Sauerstoffgehalt** | |
|---|---|---|---|---|---|---|---|---|---|---|---|---|---|---|---|
| | | **[m% FS]** | | **[m% TS]** | | **[kg/kg aschefrei]** | | | | | | | | | |
| | | Mittelw. | Stabw. | Mittelw. | Stabw. | Mittelw | Stabw. | Mittelw. | Stabw. | Mittelw. | Stabw. | Mittelw. | Stabw. | Mittelw. | Stabw. |
| **Restmüll** | **Inert** | 6,5 | 2,0 | 100,0 | 2,0 | - | - | - | - | - | - | - | - | - | - |
| | **Biogen** | 38,7 | 6,6 | 16,1 | 3,4 | 0,467 | 0,0069 | 0,067 | 0,0012 | 0,0032 | 0,0004 | 0,013 | 0,0011 | 0,446 | 0,0086 |
| | **Fossil** | 18,6 | 5,6 | 19,0 | 4,5 | 0,719 | 0,029 | 0,103 | 0,0068 | 0,003 | 0,001 | 0,015 | 0,0015 | 0,145 | 0,036 |
| **Gewerbemüll und Sperrmüll** | **Inert** | 7,6 | 1,9 | 100,0 | 2,0 | - | - | - | - | - | - | - | - | - | - |
| | **Biogen** | 20,5 | 4,3 | 11,5 | 2,4 | 0,473 | 0,007 | 0,065 | 0,0008 | 0,0037 | 0,0013 | 0,011 | 0,0033 | 0,446 | 0,0075 |
| | **Fossil** | 11,1 | 6,2 | 20,4 | 5,4 | 0,746 | 0,040 | 0,109 | 0,0093 | 0,003 | 0,0013 | 0,010 | 0,001 | 0,115 | 0,049 |

**Tabelle 7 Elementarzusammensetzung der "Stoffgruppen "für den in einer untersuchten MVA behandelten Müll (aus Sortieranglysen bestimmt)**

| | **Gruppe** | **Wassergehalt** | | **Aschegehalt** | | **Kohlenstoffgehalt** | | **Wasserstoffgehalt** | | **Schwefelgehalt** | | **Stickstoffgehalt** | | **Sauerstoffgehalt** | |
|---|---|---|---|---|---|---|---|---|---|---|---|---|---|---|---|
| | | **[m% FS]** | | **[m% TS]** | | **[kg/kg aschefrei]** | | | | | | | | | |
| | | Mittelw. | Stabw. | Mittelw. | Stabw. | Mittelw. | Stabw. | Mittelw. | Stabw. | Mittelw. | Stabw. | Mittelw. | Stabw. | Mittelw. | Stabw. |
| **System-müll** | **Inert** | 6,7 | 2,0 | 100,0 | 2,0 | 0 | 0 | 0 | 0 | 0 | 0 | 0 | 0 | 0 | 0 |
| | **Biogen** | 34,0 | 6,0 | 14,9 | 3,1 | 0,468 | 0,0069 | 0,066 | 0,0011 | 0,0033 | 0,0007 | 0,012 | 0,0016 | 0,446 | 0,0083 |
| | **Fossil** | 16,5 | 5,8 | 19,4 | 4,8 | 0,726 | 0,032 | 0,105 | 0,0075 | 0,003 | 0,0011 | 0,014 | 0,0013 | 0,137 | 0,039 |

### 2) Bestimmung der stofflichen Zusammensetzung (C-, H-, O-, N-, S-Gehalt) der fossilen Materialien über nationale bzw. übernationale Konsumdaten, Einsatzbereiche und Entsorgungswege der Polymere

Aus den Polymerkonsumdaten der Europäischen Union (EU-15) den Anwendungsgebieten der einzelnen Polymere, ihren mittleren Aufenthaltszeiten und den Entsorgungswegen lässt sich die Zusammensetzung der Kunststoffe in Siedlungsabfällen (nach Polymertyp) berechnen (Tabelle 8). Die Verwendung dieser Daten in Kombination mit der stofflichen Zusammensetzung der einzelnen Polymere erlaubt eine mittlere stoffliche Zusammensetzung der Kunststoffe in Siedlungsabfällen zu berechnen (Tabelle 9). Durch Berücksichtigung der Unsicherheiten aller Daten, lässt sich zusätzlich zu den Mittelwerten auch ein Unsicherheitsbereich für jenen Parameter ausweisen.

Eine Modellierung verschiedener Polymeranteile in Siedlungsabfällen mittels Monte-Carlo Simulation zeigt, dass zwischen dem Kohlenstoff-, dem Wasserstoff- und dem Sauerstoffgehalt eine Korrelation besteht. Diese Information kann zur Lösung des Gleichungssystems miteinbezogen werden.

**Tabelle 8 Polymer-Verbrauch an Polymeranteil in Siedlungsabfälle (EU-15)**

| **Polymere** | **Polymer-Verbrauch (PlasticsEurope, 2004)** | **Polymeranteile in Siedlungsabfällen** | |
|---|---|---|---|
| | Mittelwert | | Standardabweichung |
| | [Massen-%] | | |
| PE+PP | 48,3% | 58,6% | 8,3% |
| PET | 8,6% | 15,0% | 5,7% |
| PS, EPS | 7,0% | 6,4% | 2,7% |
| PVC | 13,1% | 4,2% | 2,2% |
| PUR | 6,1% | 3,5% | 1,6% |
| SAP | 1,1% | 3,4% | 1,2% |
| Amino | 5,9% | 2,4% | 1,5% |
| pA | 3,0% | 2,3% | 1,4% |
| ASA | 0,8% | 1,5% | 0,9% |
| Phenole | 2,2% | 0,93% | 0,57% |
| PC | 1,1% | 0,76% | 0,47% |
| PMMA | 0,7% | 0,74% | 0,45% |
| SAN | 0,5% | 0,22% | 0,13% |
| ABS | 0,5% | 0,22% | 0,13% |
| Sonstige | 1,1% | - | |
| **Summe** | **100,0%** | **100,0%** | |

**Tabelle 9 Elementarzusammensetzung der fossilen "Stoffgruppe " aus (EU-15)-Daten**

| Gruppe | Kohlenstoffgehalt | | Waserstoffgehalt | | Schwefelgehalt | | Stickstoffgehalt | | Sauerstoffgehalt | |
|---|---|---|---|---|---|---|---|---|---|---|
| | [kg/kg aschefrei] | | | | | | | | | |
| | Mittelw. | Stabw. | Mittelw | Stabw. | Mittelw. | Stabw. | Mittelw. | Stabw. | Mittelw. | Stabw. |
| Fossil | 0,768 | 0,020 | 0,109 | 0,0070 | 0,003 | 0,0011 | 0,013 | 0,005 | 0,088 | 0,022 |

Da Verbrennungsanlagen üblicherweise keine Daten über die Abfallzusammensetzung (insbesondere über die stoffliche Zusammensetzung der einzelnen Sortierfraktionen) besitzen, empfiehlt es sich, jene Berechnungsvariante zu wählen, die vorsieht, dass der Abfall in 4 Stoffgruppen unterteilt wird. Dies bedeutet, dass als fixe Stoffdaten nur Daten über den C-, H-, O-, N-, S-Gehalt der biogenen und fossilen Materialien benötigt werden.

Generell sind dazu sofern der Abfallinput der Anlage nicht direkt durch repräsentative Sortieranalysen und Elementaranalysen der Sortierfraktionen charakterisiert ist, folgende Werte (Tabelle 10) heranzuziehen:

**Tabelle 10 Elementarzusammensetzung der "Stoffgruppen " für Mischabfälle im Allgemeinen**

| Stoffgruppe | Kohlenstoffgehalt | | Wasserstoffgehalt | | Schwefelgehalt | | Stickstoffgehalt | | Sauerstoffgehalt | |
|---|---|---|---|---|---|---|---|---|---|---|
| | [kg/kg aschefrei] | | | | | | | | | |
| | Mittelw. | Stabw. | Mittelw. | Stabw. | Mittelw. | Stabw. | Mittelw. | Stabw. | Mittelw. | Stabw. |
| Biogen | 0,468 | 0,0069 | 0,066 | 0,0011 | 0,0033 | 0,0007 | 0,012 | 0,0016 | 0,446 | 0,0083 |
| Fossil | 0,768 | 0,020 | 0,109 | 0,0070 | 0,003 | 0,0011 | 0,013 | 0,005 | 0,088 | 0,022 |

### Betriebsdaten der untersuchten MVA

Im Rahmen des Routinebetriebs der untersuchten MVA werden folgende Größen gemessen, mit deren Hilfe sich der Aschegehalt, der Kohlenstoffgehalt, der Heizwert sowie die Differenz aus Sauerstoffverbrauch und Kohlendioxidproduktion der behandelten Abfälle berechnen lassen (siehe Tabellen 11-14):
- Masse des behandelten Abfalls [t FS/Monat]
- Masse sowie mittlerer Wassergehalt der festen Rückstände [t FS/Monat bzw. kg H₂0/kg FS]
- eingesetzte Menge an Zusatzbrennstoffen (Erdöl bzw, Erdgas) [t/Mo bzw. Nm³/Monat]
- Reingasmenge (trocken und auf 11 Vol-% O₂ bezogen) [Nm³/h]
- O₂ und CO₂-Konzentration im Reingas [Vol-%]
- Dampfproduktion des Kessels [t H₂0]
- Dampfdruck [bar] und Dampftemperatur [°C] im Kessel
- Speisewassertemperatur des Dampfkessels [°C].

Für die Erstellung der ursprünglich geplanten Wasserbilanz der Verbrennungsanlage (Systembild siehe Fig. 2) und der darauf aufbauenden Wassergehaltsbestimmung des Abfalls reichen die zum jetzigen Zeitpunkt zur Verfügung stehenden Betriebsdaten der Anlage nicht aus. Um eine vollständige Wasserbilanzierung durchführen zu können, müssten der Wassergehalt im Reingas und der Wasserinput in die Wäscher bekannt sein. Die ursprünglich angedachte "einfache Bestimmung" des Wassergehalts im Reingas über den Druck und die Temperatur im Wäscher 1 (unter der Annahme der Wasserdampfsättigung), scheint aufgrund der vorliegenden Messreihe nicht zulässig. Es ist über das Jahr (bis zur nächsten Revision der Anlage) eine deutliche Zunahme der Temperatur im Wäscher zu erkennen (siehe Tabelle 23). Dies deutet auf zeitabhängige Veränderungen der Bedingungen im Wäscher hin (z.B.: Ablagerungen bzw. Verschmutzungen um den Temperatursensor oder unvollständige Sättigung des Rauchgases).

Um den Wassergehalt des Abfalls bestimmen zu können, wäre daher eine erhebliche messtechnische Aufrüstung der Anlage (Wasserzähler für den Waschwasserinput und Feuchtigkeitsmessung im Reingas) erforderlich. Angestellte Überlegungen und Berechnungen zeigen jedoch, dass der zusätzliche Informationsgehalts des Wasseranteils im Abfall für den schlussendlich zu bestimmenden biomassenbürtigen Heizwertanteil bzw. die biomassebürtigen Kohlendioxidemissionen, nur gering ist.

**Tabelle 11 Behandelte Müllmassen, anfallende Rückstände und darauf basierende Aschenbilanz der untersuchten MVA (Monatswerte)**

| | **Einheit** | **Okt. 04** | **Nov. 04** | **Dez. 04** | **Jan. 05** | **Feb. 05** | **Mrz. 05** | **Apr. 05** | **Mai. 05** | **Jun. 05** | **Jul. 05** | **Aug. 05** | **Sep. 05** | **Summe bzw. Mittelwert** |
|---|---|---|---|---|---|---|---|---|---|---|---|---|---|---|
| Restmüllinenge | [to/Mo] | 14.054 | 14.233 | 15.467 | 15.340 | 12.826 | 14.676 | 14.919 | 15.324 | 14.028 | 10.176 | 14.426 | 13.693 | 169.164 |
| Sperr- und Gewerbeabfall | [to/Mo] | 0 | 0 | 0 | 0 | 0 | 0 | 0 | 0 | 0 | 0 | 0 | 0 | 0 |
| Sonstige Abfälle | [to/Mo] | 0 | 0 | 0 | 0 | 0 | 0 | 0 | 0 | 0 | 0 | 0 | 0 | 0 |
| **Gesamte Müllmenge (MW)** | **[to/Mo]** | **14.054** | **14.233** | **15.467** | **15.340** | **12.826** | **14.676** | **14.919** | **15.324** | **14.028** | **10.176** | **14.426** | **13.693** | **169.164** |
| *Gesamte Müllmenge (Stabw.) ¹⁾* | [to/Mo] | *281* | *285* | *309* | *307* | *257* | *294* | *298* | *306* | *281* | *204* | *289* | *274* | *981* |
| Zusatzbrennstoff - Erdöl | [to/Mo] | 0 | 0 | 0 | 0 | 0 | 0 | 0 | 0 | 0 | 0 | 0 | 0 | 0 |
| Zusatzbrennstoffe - Erdgas | [Nm³/Mo] | 95.520 | 35.880 | 7.620 | 28.800 | 78.060 | 16.220 | 21.380 | 4.320 | 19.780 | 28.540 | 30.460 | 83.360 | **449.940** |
| Schlacke (Wassergeh. 20 ± 4 %.) | [to/Mo] | 3.009 | 3.399 | 3.758 | 3.691 | 3.338 | 3.670 | 4.035 | 4.142 | 3.368 | 2.571 | 3.852 | 2.830 | **41.663** |
| Asche (Wassergeh. 28 ± 5 %) | [to/Mo] | 522 | 723 | 846 | 827 | 712 | 826 | 706 | 755 | 541 | 425 | 578 | 691 | 8.150 |
| Schrott | [to/Mo] | 214 | 230 | 178 | 242 | 208 | 200 | 306 | 224 | 228 | 90 | 264 | 214 | 2.598 |
| Summe der Rückstände (MW) | [to TS/Mo] | 2.997 | 3.469 | 3.793 | 3.790 | 3.391 | 3.730 | 4.043 | 4.081 | 3.312 | 2.453 | 3.762 | 2.976 | **41.797** |
| *Summe der Rückstände (Stdabw)* | [to TS/Mo] | 123 | 141 | 156 | 153 | 138 | 152 | 165 | 170 | 137 | 105 | 157 | 118 | *500* |
| **"Aschegehalt" auf FS bezogen (MW)** | **[kg TS/kg FS]** | **0,213** | **0,244** | **0,245** | **0,247** | **0,264** | **0,254** | **0,271** | **0,266** | **0,236** | **0,241** | **0,261** | **0,217** | **0,247** |
| *"Aschegehalt" auf FS bezogen (Stabw.)* | *[kg TS*//*kg FS]* | *0,009* | *0,010* | *0,010* | *0,010* | *0,011* | *0,010* | *0,011* | *0,011* | *0,010* | *0,010* | *0,011* | *0,009* | *0,003* |

| | | | | | | | | | | | | | | |
|---|---|---|---|---|---|---|---|---|---|---|---|---|---|---|
| *1) Messgenauigkeit der Waage 2%.* | | | | | | | | | | | | | | |

**Tabelle 12 Reingasmengen, Reingaskonzentrationen (O₂, CO₂) und darauf basierende Kohlenstoffbilanz der untersuchten MVA (Monatswerte)**

| | **Einheit** | **Okt. 04** | **Nov. 04** | **Dez. 04** | **Jan. 05** | **Feb. 05** | **Mrz. 05** | **Apr. 05** | **Mai. 05** | **Jun. 05** | **Jul. 05** | **Aug. 05** | **Sep. 05** | **Summe bzw. Mittelwert** |
|---|---|---|---|---|---|---|---|---|---|---|---|---|---|---|
| O₂ - Konz. im Reingas | [Vol-% trock.] | 7,74 | 8,10 | 7,85 | 7,47 | 7,80 | 7,15 | 7,06 | 6,88 | 7,49 | 7,29 | 7,55 | 7,63 | 7,50 |
| CO₂ - Konz. im Reingas | [Vol-% trock.] | 11,09 | 10,62 | 10,98 | 11,41 | 11,01 | 11,68 | 11,87 | 11,97 | 11,37 | 11,51 | 11,16 | 11,25 | 11,33 |
| Reingasmenge | [1.000 Nm³/Mo] trocken | 57.164 | 56.933 | 60.534 | 57.906 | 49.785 | 57.424 | 55.536 | 57.197 | 55.980 | 38.692 | 57.599 | 56.964 | 661.716 |
| Kohlenstoff-Input über Zusatzbrennstoffe | [to/Mo] | 45 | 17 | 4 | 14 | 37 | 8 | 10 | 2 | 9 | 13 | 14 | 39 | 213 |
| **Kohlenstoff-Output über das Reingas - gesamt (MW)** | **[to/Mo]** | **3.398** | **3.239** | **3.559** | **3.539** | **2.938** | **3.592** | **3.532** | **3.668** | **3.411** | **2.386** | **3.444** | **3.432** | **40.137** |
| *Kohlenstoff-Output über das Reingas (Stdabw.)* ^{1), 2)} | *[to*/*Mo]* | *173* | *165* | *181* | *180* | *150* | *183* | *180* | *187* | *174* | *122* | *176* | *175* | 594 |
| **Kohlenstoffgehalt des Abfalls (MW)** | **[g/kg FS]** | **243** | **230** | **234** | **234** | **230** | **249** | **240** | **244** | **247** | **237** | **242** | **252** | **240,3** |
| *Kohlenstoffgehalt des Abfalls (Stdabw.)* ^{1), 2)} | *[g*/*kg FS]* | *13* | *12* | *12* | *12* | *12* | *13* | *12* | *12* | *13* | *12* | *12* | *13* | *4* |

| | | | | | | | | | | | | | | |
|---|---|---|---|---|---|---|---|---|---|---|---|---|---|---|
| *1) Annahme: Standardabweichung der O₂- und CO₂-Messungen im Reingas: 1 % des Monatsmittelwertes* *2) Annahme: Standardabweichung der gemessenen Reingasmengen: 5 % des Monatsmittelwertes* | | | | | | | | | | | | | | |

**Tabelle 13 Dampfproduktion und darauf basierende Energiebilanz der untersuchten MVA (Monatswerte)**

| | **Einheit** | **Okt. 04** | **Nov. 04** | **Dez. 04** | **Jan. 05** | **Feb. 05** | **Mrz. 05** | **Apr. 05** | **Mai. 05** | **Jun. 05** | **Jul. 05** | **Aug. 05** | **Sep. 05** | **Summe bzw. Mittelwert** |
|---|---|---|---|---|---|---|---|---|---|---|---|---|---|---|
| Dampfproduktion ¹⁾ | [to/Mo] | 40.921 | 39.109 | 42.832 | 42.408 | 34.600 | 43.034 | 41.668 | 43.479 | 40.485 | 29.173 | 41.269 | 40.542 | 479.521 |
| Dampfdruck im Kessel | [bar] | 38 | 38 | 39 | 38 | 38 | 39 | 39 | 39 | 38 | 38 | 38 | 38 | 38,4 |
| Dampftemperatur im Kessel [°C] | [°C] | 394 | 395 | 395 | 395 | 395 | 395 | 395 | 395 | 395 | 395 | 395 | 395 | 395 |
| Speisewassertemperatur [°C] | [°C] | 126 | 124 | 123 | 117 | 121 | 121 | 121 | 121 | 121 | 122 | 118 | 117 | 121 |
| Nettoenthalpie des Wasserdampfes ²⁾³⁾ | [kJ/kg H₂O] | 2.678 | 2.687 | 2.692 | 2.716 | 2.701 | 2.701 | 2.701 | 2.700 | 2.699 | 2.694 | 2.712 | 2.716 | 2.487 |
| im Dampfkessel erzeugte Energie | [GJ/Mo] | 109.600 | 105.100 | 115.300 | 115.200 | 93.500 | 116.300 | 112.600 | 117.400 | 109.300 | 78.600 | 111.900 | 110.100 | .294.900 |
| dem Dampfkessel zugeführte gesamte Energie ⁴⁾ | [GJ/Mo] | 132.000 | 126.600 | 138.900 | 138.800 | 112.600 | 140.100 | 135.600 | 141.400 | 131.700 | 94.700 | 134.800 | 132.700 | .559.900 |
| Energieinhalt der Zusatzbrennstoffe ⁵⁾ | [GJ/Mo] | 3.420 | 1.290 | 270 | 1.030 | 2.800 | 580 | 770 | 150 | 710 | 1.020 | 1.090 | 2.990 | 16.120 |
| Energieinhalt der eingesetzten Abfälle | [GJ/Mo] | 128.600 | 125.300 | 138.600 | 137.700 | 109.800 | 139.500 | 134.800 | 141.300 | 130.900 | 93.700 | 133.700 | 129.700 | .543.600 |
| **Heizwert des Abfalls (MW)** | **[kJ/kg FS]** | **9.150** | **8.800** | **8.960** | **8.980** | **8.560** | **9.500** | **9.040** | **9.220** | **9.330** | **9.200** | **9.270** | **9.470** | **9.123** |
| *Heizwert des Abfalls (Stdabw.)* | *[kJ*/*kg FS]* | *350* | *330* | *330* | *340* | *330* | *350* | *340* | *340* | *350* | *350* | *350* | *360* | 99 |

| | | | | | | | | | | | | | | |
|---|---|---|---|---|---|---|---|---|---|---|---|---|---|---|
| *1) Standardabweichung der Dampfmengenmessung: 2 % des Monatsmittelwertes* *2) aus Speisewassertemperatur sowie Dampftemperatur und Dampfdruck (am Ende des Kessels) berechnet* *3) Standardabweichung der Netto-Enthalpie: 100 kJ*/*kg* *4) Wirkungsgrad des Dampfkessels (*η*=0,83 ± 0,02)* *5) Heizwert von Erdöl extra-leicht: 43.142 kJ*/*kg; von Erdgas: 35.838 kJ*/*m³* | | | | | | | | | | | | | | |

**Tabelle 14 Sauerstoffumsatz und Differenz aus Sauerstoffverbrauch und Kohlendioxidproduktion der untersuchten MVA (Monatswerte)**

| | **Einheit** | **Okt. 04** | **Nov. 04** | **Dez. 04** | **Jan. 05** | **Feb. 05** | **Mrz. 05** | **Apr. 05** | **Mai. 05** | **Jun. 05** | **Jul. 05** | **Aug. 05** | **Sep. 05** | **Summe bzw. Mittelwert** |
|---|---|---|---|---|---|---|---|---|---|---|---|---|---|---|
| Reingasmenge ¹⁾ | [1.000 Nm³/Mo] trocken | 57.164 | 56.933 | 60.534 | 57.906 | 49.785 | 57.424 | 55.536 | 57.197 | 55.980 | 38.692 | 57.599 | 56.964 | 661.716 |
| O₂ - Konz. im Reingas | [Vol-% trock.] | 7,74 | 8,10 | 7,85 | 7,47 | 7,80 | 7,15 | 7,06 | 6,88 | 7,49 | 7,29 | 7,55 | 7,63 | 7,50 |
| CO₂ - Konz. im Reingas | [Vol-% trock.] | 11,09 | 10,62 | 10,98 | 11,41 | 11,01 | 11,68 | 11,87 | 11,97 | 11,37 | 11,51 | 11,16 | 11,25 | 11,33 |
| Summe O₂+CO₂ im Reingas (MW) | [Vol-% trock.] | 18,8 | 18,7 | 18,8 | 18,9 | 18,8 | 18,8 | 18,9 | 18,9 | 18,9 | 18,8 | 18,7 | 18,9 | 18,83 |
| *Summe O₂+CO₂ im Reingas (Stdabw.)* ¹⁾ | *[Vol-% trock.]* | *0,1* | *0,1* | *0,1* | *0,1* | *0,1* | *0,1* | *0,1* | *0,1* | *0,1* | *0,1* | *0,1* | *0,1* | *0,04* |
| **Sauerstoffumsatz (MW)** | **[mol/kg FS]** | **24,5** | **23,8** | **23,8** | **23,5** | **23,3** | **25,0** | **23,9** | **24,4** | **24,9** | **24,0** | **24,8** | **25,3** | **24,26** |
| *Sauerstoffumsatz (Stdabw.)* | *[mol*/*kg FS]* | *1,3* | *1,2* | *1,2* | *1,2* | *1,2* | *1,3* | *1,2* | *1,2* | *1,3* | *1,2* | *1,3* | *1,3* | *0,4* |
| **Differenz aus Sauerstoffverbrauch und Kohlendioxidproduktion (MW)** | **[mol/kg FS]** | **4,7** | **. 5,0** | **4,7** | **4,4** | **4,5** | **4,7** | **4,2** | **4,5** | **4,7** | **4,6** | **5,0** | **4,7** | **4,65** |
| *Differenz aus Sauerstoffverbrauch und Kohlendioxidproduktion (Stdabw.)* | *[mol*/*kg FS]* | *0,40* | *0,40* | *0,38* | *0,37* | *0,38* | *0,39* | *0,36* | *0,37* | *0,39* | *0,37* | *0,40* | *0,40* | *0,11* |

| | | | | | | | | | | | | | | |
|---|---|---|---|---|---|---|---|---|---|---|---|---|---|---|
| *1) Annahme: Standardabweichung der gemessenen Reingasmengen: 5 % des Monatsmittelwertes* *2) Annahme: Standardabweichung der O₂- und CO₂-Messungen im Reingas: 1 % des Monatsmittelwertes* | | | | | | | | | | | | | | |

### Resultate

### Ausgleichsrechnung

### Analyse des Gleichungssystems und Aussagekraft der Ergebnisse

### Exkurs lineare Gleichungssysteme:

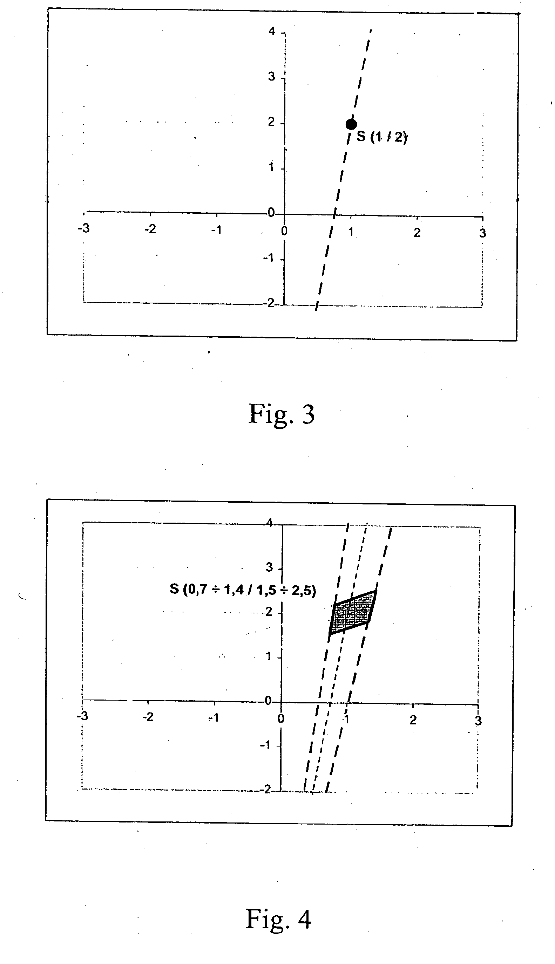

Die Lösung von linearen Gleichungssystetmen mit drei Unbekannten kann man sich vereinfacht als Schnittpunkt von mehreren Ebenen vorstellen. Eine exakte Lösung (ein Schnittpunkt) einen solchen Systems ist dann möglich, wenn das System weder unterbestimmt noch überbestimmt ist (drei Unbekannte in drei Gleichungen). Zur Veranschaulichung ist in Fig. 3 die Lösung eines "einfach bestimmten" Gleichungssystems mit zwei Unbekannten dargestellt (Schnittpunkt zweier Geraden).

Lineares Gleichungssystem mit zwei Unbekannten: $- 0 , 5 ⁢ x + y = 1 , 5$ $- 8 , 0 ⁢ x + y = - 6$

Sind die Koeffizienten der Unbekannten des Gleichungssystems nicht exakt gegeben sondern durch Mittelwerte und deren Unsicherheiten so stellt sich die Lösung folgendermaßen dar: $\left(-0,5\pm 0,05\right) ⁢ x + y = 1 , 5 \pm 0 , 25$ $\left(-8,0\pm 1,60\right) ⁢ x + y = - 6 \pm 0 , 50$

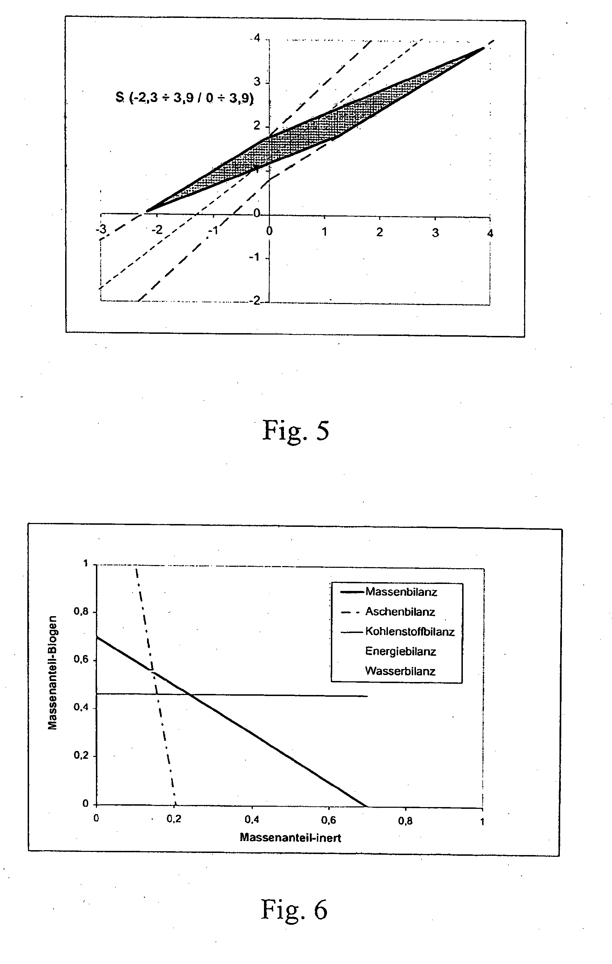

Als Lösung erhält man hier keinen Schnittpunkt sondern eine Schnittfläche (siehe Fig. 4). Die Größe der Schnittfläche bzw. die Differenz der Eckpunktkoordinaten der Fläche (repräsentativ für die Unsicherheit des Resultats) hängt einerseits von der angegebene Unsicherheit der Koeffizienten ab und andererseits von der Lage der "Geraden" zueinander. Je schleifender der Schnitt der Geraden (d.h. je ähnlicher die Koeffizienten der Gleichungen sind) ist, desto "unsicherer" ist das Resultat (siehe Fig. 5).

Bei überbestimmten Gleichungssystemen besitzen daher jene Gleichungen mit sehr ähnlichen Koeffizienten (schleifender Schnitt!) nur einen geringen Informationsgehalt für das Resultat. Werden Gleichungen mit mehr als zwei Unbekannten miteinander "verschnitten", so ist das Ergebnis für jene Unbekannten mit sehr ähnlichen Koeffizienten relativ ungenau, hingegen weist das Ergebnis für die Unbekannten mit stark unterschiedlichen Koeffizienten geringe Unsicherheiten auf.

Im Folgenden wurde versucht basierend auf den oben angestellten Überlegungen die Bilanzgleichungen (Massenbilanz, Aschebilanz, Kohlenstoffbilanz, Energiebilanz, Sauerstoffverbrauchs- und Kohlendioxidproduktions-Bilanz" und Wasserbilanz) auf ihren Informationsgehalt für das Endresultat im Fall von 3 Unbekannten Stoffgruppen (Masse an inerten (m_{I}), an biogenen (m_{B}) und an fossilen (m_{F}) Materialien) zu untersuchen. Es wurden dazu die Koeffizienten der Unbekannten in den einzelnen Gleichungen mit einander verglichen.

### In Gleichung 1 sind die sechs Bilanzgleichungen zusammengestellt. Die Koeffizienten des Gleichungssystems sind durch die Mittelwerte ("wahrscheinlichste Werte) und deren Unsicherheitsbereiche (Standardabweichung) definiert. Sie wurden aus der Elementarzusammensetzung der Stoffgruppen (siehe Tabelle 7) errechnet. Die "konstanten Faktoren" auf der rechten Seite der Gleichungen werden durch die Betriebsdaten der Anlage vorgegeben. Aufgrund fehlender Information hinsichtlich der Wasserbilanz der Anlage musste (hier im Rahmen der Analyse des Gleichungssystems) für die "Konstante" dieser Gleichung ein wahrscheinlichster Näherungswert mit dazugehörigem größerem Unsicherheitsbereich angenommen werden. Gleichung 1 Bilanzgleichungen (in der Reihenfolge: Massen-, Aschen-, Kohlenstoff , Energie-, "Sauerstoffverbrauchs- und Kohlendioxidproduktions-" und Wasserbilanz)

$\begin{matrix}\begin{matrix}{m}_{I} + & {m}_{B} + & {m}_{F} & = 1 \\ \left(0,933\pm 0,021\right) ⁢ {m}_{I} + & \left(0,098\pm 0,037\right) ⁢ {m}_{B} + & \left(0,162\pm 0,061\right) ⁢ {m}_{F} & = 0 , 24 \pm 0 , 01 \\ & \left(263\pm 26\right) ⁢ {m}_{B} + & \left(489\pm 50\right) ⁢ {m}_{F} & = 254 \pm 27 \\ \left(-164\pm 49\right) ⁢ {m}_{I} + & \left(9.180\pm 1.030\right) ⁢ {m}_{B} + & \left(22.300\pm 1.900\right) ⁢ m & = 11.100 \pm 790 \\ & \left(2,00\pm 0,70\right) ⁢ {m}_{B} + & \left(15,40\pm 1,47\right) ⁢ {m}_{F} & = 4 , 27 \pm 0 , 70 \\ \left(0,067\pm 0,02\right) ⁢ {m}_{I} + & \left(0,675\pm 0,178\right) ⁢ {m}_{B} + & \left(0,799\pm 0,155\right) ⁢ {m}_{F} & = 0 , 60 \pm 0 , 20\end{matrix}\end{matrix}$

Um die Koeffizienten der Unbekannten (m_{I}, m_{B} und m_{F}) besser vergleichen zu können, wurde eine Normierung der Gleichungen durchgeführt, so dass der Koeffizient für den Biomasseanteil in jeder Gleichung 1 beträgt (siehe Gleichung 2)

### Gleichung 2 normierte Bilanzgleichungen (in der Reihenfolge: Massen-, Aschen-, Kohlenstoff-, Energie-, "Sauerstoffverbrauchs- und Kohlendioxidproduktions-" und Wasserbilanz)

$\begin{matrix}\begin{matrix}{m}_{I} + & {m}_{B} + & {m}_{F} & = 1 \\ \left(9,49\pm 0,43\right) ⁢ {m}_{I} + & \left(1,00\pm 0,38\right) ⁢ {m}_{B} + & \left(1,65\pm 0,62\right) ⁢ {m}_{F} & = 2 , 45 \pm 0 , 1 \\ & \left(1,00\pm 0,099\right) ⁢ {m}_{B} + & \left(1,86\pm 0,19\right) ⁢ {m}_{F} & = 0 , 97 \pm 0 , 10 \\ \left(-0,018\pm 0,005\right) ⁢ {m}_{I} + & \left(1,00\pm 0,11\right) ⁢ {m}_{B} + & \left(2,43\pm 0,21\right) ⁢ m & = 1 , 21 \pm 0 , 086 \\ & \left(1,00\pm 0,35\right) ⁢ {m}_{B} + & \left(7,70\pm 0,74\right) ⁢ {m}_{F} & = 2 , 14 \pm 0 , 35 \\ \left(0,10\pm 0,03\right) ⁢ {m}_{I} + & \left(1,00\pm 0,26\right) ⁢ {m}_{B} + & \left(1,18\pm 0,23\right) ⁢ {m}_{F} & = 0 , 89 \pm 0 , 27\end{matrix}\end{matrix}$

Ein Vergleich der Koeffizienten für den inerten Massenanteil m_{I} zeigt, dass diese Faktoren in den Gleichungen: Kohlenstoff-, Energie-, "Sauerstoffverbrauchs- und Kohlendioxidproduktions-" und Wasserbilanz relativ ähnlich (im Bereich von Null) sind. Eine alleinige Kombination dieser Gleichungen würde daher zu einem sehr unsicheren Resultat (inerter Massenanteil) führen (schleifender Schnitt der "Ebenen" - Fig. 6). Die Koeffizienten für die restlichen beiden Gleichungen (Massen- und Aschenbilanz) unterscheiden sich jedoch deutlich (1 bzw. 9,49). Durch Kombination von Massen-, Aschen- und beispielsweise Energiebilanz (oder Wasserbilanz) kann somit der Inertanteil m_{I} relativ exakt bestimmt werden (guter Schnittwinkel der "Ebenen" - Fig. 6).

Die Koeffizienten für den fossilen Massenanteil m_{F} liegen im Gleichungssystem im Bereich von 1 bis 7,70. Hier gilt ebenso, dass eine Kombination von Gleichungen mit möglichst unterschiedlichen Koeffizienten zu einem exakteren Ergebnis führt. Die Tatsache, dass die Koeffizienten von m_{F} in der Massen- und Wasserbilanzgleichung nahezu identisch sind (1 bzw. 1,18), bedeutet, dass eine Einbeziehung der Wasserbilanz keine zusätzliche (wertvolle) Information für die Ermittlung des fossilen bzw. biogenen Massenanteils m_{F} bzw. m_{B} bringen würde (schleifender Schnitt der Ebenen!!). Eine Aufrüstung der Messeinrichtungen der untersuchten MVA zur vollständigen Erfassung aller Wasserströme und einer damit mögliche Wasserbilanzierung, würde daher zu keinem exakteren Ergebnis hinsichtlich des Biomasseanteils und dem in weiterer Folge zu bestimmenden biomassebürtigen Heizwertanteils (bzw. biomassebürtigen Kohlendioxidemissionen) führen.

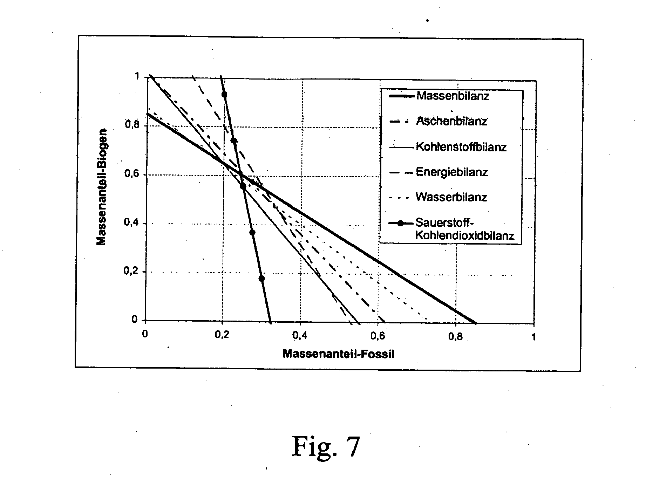

Generell liegen die Koeffizienten für den fossilen Massenanteil m_{F} mit Ausnahme der Sauerstoffverbrauchs-Kohlendioxidproduktions-Differenz-Gleichung in einem relativ engen Bereich (1 bis 2,44). Es kommt daher zu eher schleifenden Schnitten der "Ebenen" (siche Fig. 7). Das Ergebnis für m_{F} und m_{B} ist daher im Vergleich zum inerten Massenanteil m_{I} mit etwas größeren Unsicherheiten behaftet.

### Ergebnisse für konkrete MVA unter Verwendung der Variante mit 4 Stoffgruppen

Die Ausgleichsrechnung zur Lösung des aufgestellten Gleichungssystems wurde mit der unter "Mathematische Lösung der Bilanzgleichungen" beschriebenen Methode durchgeführt. Als Eingangsparameter für die Berechnung wurden die Koeffizienten aus Tabelle 10 und die berechneten Betriebsgrößen der MVA (Aschegehalt, Kohlenstoffgehalt, Heizwert der behandelten Abfälle und die Differenz aus Sauerstoffverbrauch und Kohlendioxidproduktion siehe Tabelle 11 bis 14) herangezogen. Die Ergebnisse der Ausgleichsrechnung, die Massenanteile, die Heizwertanteile sowie die Kohlenstoffanteile der drei Stoffgruppen "Inert, Biogen, Fossil und Wasser" sind in Tabelle 15 zusammengefasst.

Die Resultate zeigen, dass der biomassebürtige Heizwertanteil sowie der biogene Kohlenstoffgehalt über das Jahr nicht konstant sind, sondern erhebliche Schwankungen aufweisen. Diese Schwankungen lassen sich teilweise auf den unterschiedlichen Einsatz von Haus- und Gewerbemüll sowie auch auf jahreszeitliche Änderungen der Müllzusammensetzung zurückführen.

**Tabelle 15 Massen-, Heizwertanteil und Kohlenstoffanteile der Stoffgruppen Inert, Biogen, Fossil und Wasser über das Geschäftsjahr 2004/05**

| | | **Einheit** | **Okt. 04** | **Nov. 04** | **Dez. 04** | **Jan. 05** | **Feb. 05** | **Mrz. 05** | **Apr. 05** | **Mai. 05** | **Jun. 05** | **Jul. 05** | **Aug. 05** | **Sep. 05** |
|---|---|---|---|---|---|---|---|---|---|---|---|---|---|---|
| **Massenanteil** | Biogen | [kg/kg FS] | 0,283 | 0,216 | 0,252 | 0,284 | 0,258 | 0,294 | 0,312 | 0,301 | 0,290 | 0,280 | 0,247 | 0,304 |
| | Fossil | | 0,152 | 0,176 | 0,160 | 0,143 | 0,147 | 0,152 | 0,130 | 0,141 | 0,152 | 0,151 | 0,171 | 0,149 |
| | Inert | | 0,213 | 0,244 | 0,246 | 0,248 | 0,265 | 0,255 | 0,271 | 0,267 | 0,236 | 0,242 | 0,261 | 0,218 |
| | Wasser | | 0,352 | 0,364 | 0,342 | 0,326 | 0,330 | 0,299 | 0,287 | 0,292 | 0,322 | 0,328 | 0,321 | 0,329 |
| **Heizwertanteil *** | Biogen* | [%] | 46,3% | 36,3% | 42,1% | 47,8% | 44,8% | 47,2% | 52,6% | 49,7% | 46,9% | 46,1% | 40,1% | 48,5% |
| | Fossil | | 53,7% | 63,7% | 57,9% | 52,2% | 55,2% | 52,8% | 47,4% | 50,3% | 53,1% | 53,9% | 59,9% | 51,5% |
| | *Stdabw* | | 6,5% | 6,5% | 6,5% | 6,4% | 6,5% | 6,3% | 6,3% | 6,3% | 6,41% | 6,4% | 6,4% | 6,5% |
| **Energieproduktion Dampfkessels** | Mittelwert | [GJ/Mo] | 128.626 | 125.320 | 138.639 | 137.744 | 109.802 | 139.480 | 134.847 | 141.282 | 130.950 | 93,670 | 133.738 | 129.668 |
| **Kohlenstoffanteile** | Biogen | [kg/kg FS] | 0,132 | 0,101 | 0,118 | 0,133 | 0,121 | 0,138 | 0,146 | 0,141 | 0,136 | 0,131 | 0,116 | 0,142 |
| | *Stdabw* | | 0,017 | 0,017 | 0,017 | 0,017 | 0,016 | 0,017 | 0,017 | 0,017 | 0,017 | 0,017 | 0,017 | 0,018 |
| | Fossil | | 0,117 | 0,135 | 0,123 | 0,110 | 0,113 | 0,117 | 0,100 | 0,108 | 0,117 | 0,116 | 0,131 | 0,115 |
| | *Stdabw* | | 0,016 | 0,016 | 0,015 | 0,015 | 0,015 | 0,015 | 0,015 | 0,015 | 0,015 | 0,015 | 0,016 | 0,016 |
| **Verbrannte Abfallmasse (korrigierte Werte)** | Mittelwert | [to/Mo] | 13.963 | 14.139 | 15.352 | 15.209 | 12.762 | 14.581 | 14.825 | 15.228 | 13.943 | 10.086 | 14.341 | 13.617 |
| | *Stdabw* | | 266 | 269 | 292 | 289 | 243 | 277 | 282 | 289 | 265 | 192 | 272 | 259 |
| **Kohlenstofffracht Abfälle** | Biogen | | 1.850 | 1.434 | 1.813 | 2.021 | 1.540 | 2.008 | 2.169 | 2.146 | 1.891 | 1.321 | 1.662 | 1.937 |
| | *Stdabw* | | 247 | 243 | 263 | 257 | 213 | 256 | 250 | 258 | 245 | 173 | 250 | 247 |
| | Fossil | | 1.627 | 1.908 | 1.892 | 1.675 | 1.444 | 1.708 | 1.479 | 1.649 | 1.627 | 1.172 | 1.882 | 1.563 |
| | *Stdabw* | | 221 | 223 | 237 | 228 | 191 | 227 | 218 | 228 | 218 | 154 | 227 | 219 |
| **Kohlenstofffracht Zusatzbrennstoffe** | Mittelwert | | 45 | 17 | 4 | 14 | 37 | 8 | 10 | 2 | 9 | 13 | 14 | 39 |

| | | | | | | | | | | | | | | |
|---|---|---|---|---|---|---|---|---|---|---|---|---|---|---|
| *• biogener Heizwertanteil mithilfe empirisch abgeleiteter Formel (HW_{biogen,corr}=0,994*HW_{biogen}-0,013) korrigiert um zu berücksichtigen, dass biogene Materialien tendenziell feuchter sind als fossile Materialien* | | | | | | | | | | | | | | |

### Biomassebürtiger Anteil am Heizwert

Aus den Ergebnissen der Ausgleichsrechnungen, den Massenanteilen der vier Stoffgruppen (Inert, Biogen, Fossil und Wasser), lässt sich unter Berücksichtigung der monatlichen Energieproduktion und der errechneten monatlichen Heizwertanteile der mittlere jährliche biomassebürtige Heizwertanteil des behandelten Abfalls berechnen.

Für die Energieproduktion wurde dabei der Mittelwert aus den monatlichen biomassebürtigen Heizwertanteilen (gewichtet mit der Energieproduktion) gebildet (Daten aus Tabelle 15). Dieser Wert liegt für das Geschäftsjahr 2004/05 (Oktober 2004 bis September 2005) bei 45,8 %.

Dieser Rechenwert (45,8 %) ist anteilsmäßig um jene Energiemenge zu reduzieren, die über fossile Zusatzbrennstoffe (Erdöl bzw. Erdgas) einbracht wurde. Dieser Anteil liegt für den betrachteten Zeitraum bei rund 1,0 % der erzeugten Energie. Somit ergibt sich für das Geschäftsjahr 2004/05 (Oktober 2004 bis September 2005) ein Heizwertanteil der Biomasse von 45,3 % (siehe Tabelle 16). Die Unsicherheit dieses Ergebnisses liegt unter Berücksichtigung aller Faktoren bei rund 1,8 % (absoluter Fehler).

**Tabelle 16 Eingesetzte Energieträger (im Geschäftsjahr 2004/05)**

| **Energieträger** | **Erzeugte Wärmemenge** | | | |
|---|---|---|---|---|
| | **Gesamt [GJ/a]** | | **Anteil [%]** | |
| | **MW** | ***SD*** | **MW** | ***SD*** |
| Biomasse | 706.500 | *28.700* | **45,3%** | **1,8%** |
| Zusatzbrennstoffe (Erdgas + Erdöl) | 16.100 | - | **1,0%** | - |
| Sonstige fossile Energieträger (Kunststoffe im Müll) | 837.200 | *28. 700* | ***53,7%*** | *1,8%* |
| **Summe** | ***1.559.800*** | | ***100,0%*** | |

### Biogene und fossile Kohlendioxidemissionen

Analog zu den Heizwertanteilen lässt sich aus den Ergebnissen der Ausgleichsrechnung eine gesamte biogene Kohlenstofffracht bzw. Kohlendioxidfracht und eine fossile Kohlendioxidfracht berechnen. Unter Berücksichtigung aller Faktoren ergibt sich für das Geschäftsjahr 2004/05 folgende Verteilung:

**Tabelle 17 Kohlendioxidemissionen (im Geschäftsjahr 2004/05)**

| **Energieträger** | **Kohlendioxidemissionen** | |
|---|---|---|
| | **[to/a]** | |
| | **MW** | ***SD*** |
| Biomasse | 79.900 | *3.100** |
| Zusatzbrennstoffe (Erdgas + Erdöl) | 800 | - |
| Sonstige fossile Energieträger (Kunststoffe im Müll) | 72.000 | *2.800** |
| Summe | *152.700* | |

| | | |
|---|---|---|
| ** Anmerkung: Durch die Überbestimmtheit des Gleichungssystems verringert sich im Rahmen der Ausgleichsrechnung die ursprünglich angenommene Unsicherheit der Rahmen der Ausgleichsrechnung die ursprünglich angenommene Unsicherheit der gemessenen Betriebsparameter (z.B.: Unsicherheit der Reingasmengenmessung) deutlich, was wiederum mit einer reduzierten Unsicherheit des gesuchten Endergebnisses (z.B.: fossile Kohlendioxidemissionen) verbunden ist.* | | |

### Untersuchungen zur Stabilität des Resultates (bei Berechnung mit vier Stoffgruppen)

Um die Stabilität bzw. Robustheit der entwickelten Methode (Ausgleichsrechnung der aufgestellten Bilanzgleichungen) zu überprüfen, wurden Betriebsdaten (Heizwert, Kohlenstoffgehalt und Aschegehalt) bei unterschiedlicher Müllzusammensetzung generiert. In weiterer Folge wurde untersucht, inwieweit mit Hilfe des entwickelten Ansatzes die Unterschiede in der Müllzusammensetzung erkennbar sind. Ausgehend von den Ergebnissen der Sortieranalyse von Hauer (2000) wurde im 1. Fall (hoher Anteil an biogenen Materialien) der Anteil an Papier und Biogenem im Müll deutlich erhöht, während im 2. Fall (hoher Anteil an fossilen Materialien) der Anteil an Kunststoff vergrößert wurde (siehe Tabelle 18).

**Tabelle 18 Veränderungen der Abfallzusammensetzung gegenüber Hauer, 1999 (siehe Tabelle 2 und 3)**

| **Fraktion** | **1. Fall** | | **2. Fall** | |
|---|---|---|---|---|
| | **Veränderungen [m%] der Zusammensetzung gegenüber Hauer (1999)** | | | |
| | **Restmüll** | **Gewerbemüll** | **Restmüll** | **Gewerbemüll** |
| Papier | +12 | +18 | -7 | - |
| Holz | - | - | -2 | -14 |
| Biogenes | +5 | - | -8 | - |
| Kunststoffe | -17 | -18 | +17 | +14 |

**Tabelle 19 Generierte Betriebsdaten bei gezielt veränderter Abfallzusammensetzung**

| **Parameter** | **1. Fall** | **2. Fall** |
|---|---|---|
| Heizwert [kJ/kg FS] | 8.250 ± 700 | 13.700 ± 800 |
| Kohlenstoffgehalt [g/kg FS] | 240 ± 20 | 335 ± 30 |
| Aschegehalt [g/kg FS] | 270 ± 10 | 290 ± 10 |
| O2-Verbrauch [mol/kg FS] | 23,2 ± 2,5 | 36,5 ± 4,0 |
| O2-Verbrauch-CO2-Produktion [mol/kg FS] | 3,2 ± 0,35 | 8,7 ± 0,9 |

Der über die Elementarzusammensetzung (Formel nach Boie, 1957) berechnete biomassebürtige Heizwertanteil liegt für die veränderte Abfallzusammensetzung im Fall 1 bei 67,7 % und im Fall 2 bei 19,7 %.

Mit Hilfe der generierten Betriebsdaten (aus Tabelle 19) und den Koeffizienten der Bilanzgleichungen (aus Tabelle 7) wurden über die Ausgleichsrechnung folgende Stoffgruppenzusammensetzungen (Massenanteil inert, biogen und fossil) ermittelt:

**Tabelle 20 Vergleich der vorgegebenen Massen-, Heizwertanteile und Kohlenstoffgehalte(aus der Abfallzusammensetzung) mit jener die über die Ausgleichsrechnung bestimmt wurden**

| **Massenanteil** | **1. Fall** | | **2. Fall** | |
|---|---|---|---|---|
| | ***aus der Abfallzusammensetzung gegeben (Vorgabe)*** | **über Ausgleichsrechnung bestimmt (Berechnung)** | ***aus der Abfallzusammensetzung gegeben (Vorgabe)*** | **über Ausgleichsrechnung bestimmt (Berechnung)** |
| **Massenanteile + Heizwert** | | | | |
| Inert [m%] | *27,2* | 27,0 | *28,8* | 29,0 |
| Wasser [m%] | *26,3* | 26,8 | *19,2* | 18,9 |
| Biogen [m%] | *36,9* | 37,5 | *17,7* | 16,9 |
| Fossil [m%] | *9,6* | 8,7 | *34,3* | 35,2 |
| **Biomassebürtiger Heizwertanteil [%]** | ***67,7*** | **68,3** | ***19,7*** | **18,6** |

| **Kohlenstoffanteile** | | | | |
|---|---|---|---|---|
| **Biogen [m%]** | ***72,2*** | **73,5** | ***24,6*** | **23,3** |
| **Fossil [m%]** | ***27,8*** | **26,5** | ***75,4*** | **76,7** |

Ein Vergleich der vorgegebenen Massenanteile mit den über die Ausgleichsrechnung bestimmten Massenanteilen zeigt, dass die entwickelte Methode geeignet ist, um unterschiedliche Abfallzusammensetzungen zu erkennen und zu bestimmen. Für den inerten Massenanteil m_{I} ist der Fehler der Methodik am geringsten (< 0,25 %). Für die Massenanteile von m_{B}, m_{F} und m_{w} ist die Abweichung zwischen vorgegebenen und berechneten Werten etwas größer. Der Fehler liegt in den beiden oben dargestellten Fällen bei etwa 0,9 %.

Für das zu ermittelnde Endergebnis (den biomassebürtigen Heizwertanteil) beträgt der maximale Fehler etwa 1,1 % (Fall 1: Vorgabe: 67,7 %, Rechnung: 68,3 %; Fall 2: Vorgabe: 19,7 %, Berechnung: 18,6 %).

Gilt es die Kohlendioxidemissionen (Kohlenstoffgehalte) dem biogenen bzw. fossilen Ursprung zu zuordnen, so liegt der Fehler bei rund 1,3 % (Fall 1: Vorgabe: 72,2%, Rechnung: 73,5 %; Fall 2: Vorgabe: 24,6 %, Berechnung: 23,3 %).

Die hier ausgewiesenen Fehler beziehen sich ausschließlich auf die angewandte Methodik der Ausgleichsrechnung für die verwendeten Bilanzgleichungen. Die tatsächliche Unsicherheit (Fehler) der entwickelten Methode bei der Verwendung von realen Betriebsdaten ist größer als die oben ausgewiesenen 1,3 %.

### Zusammenfassung

Es wurde eine neue Methode zur Bestimmung des Stromanteils aus Biomasse bei der Stromauskopplung einer Müllverbrennungsanlage entwickelt. Das erfindungsgemäße Konzept eignet sich ebenso zur Bestimmung der biogenen bzw. fossilen Kohlendioxidemissionen bei der Müllverbrennung. Der Ansatz beruht auf einem Abgleich "theoretischer" Gleichungen mit gemessenen Betriebsdaten der Müllverbrennungsanlagen.

Für die theoretischen Gleichungen werden die Abfälle in drei oder vier Stoffgruppen: (Inert, Biogen und Fossil bzw. Inert, Biogen, Fossil und Wasser) unterteilt. Für diese Stoffgruppen wurden über Literaturdaten und über Ergebnisse aus Sortieranalysen mittlere stoffliche Zusammensetzungen errechnet.

Diese stoffliche Zusammensetzung der Stoffgruppen kann verwendet werden, um mit Hilfe theoretischer Gleichungen verschiedene Eigenschaften (Aschegehalt, Heizwert, Kohlenstoffgehalt, Sauerstoffumsatz, Differenz aus Sauerstoffverbrauch und Kohlendioxidproduktion) der behandelten Abfälle zu berechnen. Die theoretisch ermittelten Eigenschaften der Abfälle müssen mit den über die Betriebsdaten der Müllverbrennungsanlage ermittelten Eigenschaften übereinstimmen. Diese Übereinstimmung zwischen theoretischen und gemessenen Eigenschaften wird durch Variieren der Massenanteile der drei bzw. vier Stoffgruppen (inert, biogen und fossil bzw. inert, biogen, fossil und Wasser) erreicht. Diese Variation der Massenanteile (so dass alle Gleichungen erfüllt werden) erfolgt durch eine mathematische Ausgleichsrechnung eines linearen oder nicht linearen Gleichungssystems. Das Ergebnis der Ausgleichsrechnung sind die Massenanteile der drei bzw. vier Stoffgruppen. Mithilfe dieser Massenanteile und deren mittlerer Elementarzusammensetzung lässt sich unter Verwendung von so genannten Heizwertformeln (z.B.: Boie, 1957) der gesamte Heizwert des Abfalls und der biomassebürtiger Anteil des Heizwertes berechnen. Letzterer entspricht dem erzeugten Stromanteil aus Biomasse, der zu bestimmenden Größe.

Mit Hilfe dieser neuen Methode wurde für die untersuchte MVA für das Geschäftsjahr 2004/05 (Oktober 2004 bis September 2005) der Stromanteil aus Biomasse berechnet. Für das Geschäftsjahr 2004/05 wurde auf diese Weise ein mittlerer biomassebürtiger Heizwertanteil und damit ein Stromanteil aus Biomasse von 45,3 % errechnet. Die Unsicherheit des Ergebnisses liegt bei 1,8 %.

Die biogenen Kohlendioxidemissionen liegen für das untersuchte Geschäftsjahr bei rund 79.900±3.100 t, während im selben Zeitraum etwa 72.800±2.800t t fossiles CO₂ (aus Abfällen und Zusatzbrennstoffen) emittiert wurde.

### Literatur

Beiz, W. and Küttner, K.-H., 1986. Taschenbuch für den Maschinenbau. Springer-Verlag Berlin, Heidelberg, New York, Tokyo.
Boie, W., 1957. Vom Brennstoff zum Rauchgas. B.G. Teubner Verlagsgesellschaft, Leipzig.
Dehoust, G., Gebhardt, P. and Gärtner, S., 2002. Der Beitrag der thermischen Abfallbehandlung zu Klimaschutz, Luftreinhaltung und Ressourcenschonung, Institut für Angewandte Ökologie, Darmstadt.
Döberl, G., Huber, R., Fellner, J., Cencic, O. and Brunner, P.H., 2002. Neue Strategien zur Nachsorge von Deponien und zur Sanierung von Altlasten (Projekt STRANDEZA), Abteilung Abfallwirtschaft und Stoffhaushalt, Technische Universität Wien.
Echte, A., 1993. Handbuch der Technischen Polymerchemie. VCH Verlagsgesellschaft, Weinheim.
EDANA, 2001. Diapers - Health Benefits and Environmental Aspects, European Disposables and Nonwovens Association, Brussels.
Kost, T., 2001. Brennstofftechnische Charakterisierung von Haushaltsabfällen. Dissertation Thesis, Technische Universität Dresden, Dresden.
Morf, L., Reil, E., Ritter, E. (2004) "Vier Jahre routinemäßiges Stoffflussmonitoring an der MVA Spittelau - Resultate und Erfahrungen aus der Praxis" DEPOTECH 2004 in Leoben, 24.-26. November 2004.
Narasimhan, S., Jordache C., 2000. Data Reconciliation & Gross Error Detection. Gulf Publishing Company, Houston, Texas.
PlasticsEurope, 2004. An analysis of plastics consumption and recovery in Europe, PlasticsEurope, Bruxelles.
Schachermayer, E., Bauer, G., Ritter, E. and Brunner, P.H., 1994. Messung der Güter- und Stoffbilanz einer Müllverbrennungsanlage. 56, Umweltbundesamtes Wien GmbH., Wien.
Scholz, R., Beckmann, M. and Schulenburg, F., 2001. Abfallbehandlung in thermischen Verfahren Verbrennung, Vergasung, Pyrolyse, Verfahrens- und Anlagenkonzepte. Teubner, Stuttgart, 460 pp.
Skutan, S. and Brunner, P.H., 2003. Stoffbilanzen mechanisch-biologischer Anlagen zur Behandlung von Restmüll (SEMBA) - 5. Zwischenbericht, Technische Universität Wien, Institut für Wassergüte und Abfallwirtschaft, Wien.
TBHauer, 1999. Oberösterreichische Abfallanalysen, Klosterneuburg.
TBHauer, 2000. Bestimmung der Heizwertanteile fossiler und regenerativer Energieträger im Müll. Klostemeuburg
TBHauer, 2002. Gutachten über Art und Zusammensetzung der eingesetzten Brennstoffe, des biogenen Anteils und des Anteils an "Abfall mit hohem biogenen Anteil", Klostemeuburg.
Würdinger, E., Wagner, J., Tränkler, J. and Rommel, W., 1997. Energetische Nutzung der Biomassenanteile in Abfällen, Bayerisches Institut für Abfallforschung (BIfA GmbH), Augsburg.

### Verwendete Formelzeichen

- M_{I}, M_{B}, M_{F}: Masse der Fraktion Inert, Biogen und Fossil [kg FS]
- M_{AbfaH}: Masse des behandelten Abfalls [kg FS]
- m_{I}, m_{B}, m_{F}, m_{w}: Massenanteil der Fraktion Inert, Biogen, Fossil und Wasser [kg/kg FS]
- M_{Schrott}, M_{Schlacke}: Masse des Schrotts bzw. der Schlacke [kg TS]
- M_{EF-Asche}, M_{Kesselasche}: Masse der Elektrofilterasche bzw. Masse der Kesselasche [kg TS]
- M_{Erdgas}: Masse des zugefeuerten Erdgases bzw. Erdöls [kg]
- w_{I}, w_{B}, W_{F}: Wassergehalt der Fraktion Inert, Biogen und Fossil [kg H₂0/kg FS]
- w_{A}: Wassergehalt des Abfalls [kg H₂0/kg FS]
- w_{H}: durch Oxidation von Wasserstoff produziertes Wasser pro kg Abfall [kg H₂0/kg FS]
- W_{S}: "scheinbarer Wassergehalt" des Abfalls [kg H₂0/kg FS]
- a_{I}, a_{B}, a_{F}: Aschegehalt der Fraktion Inert, Biogen und Fossil [kg Asche/kg TS]
- a_{A}: Aschegehalt des Abfalls [kg Asche/kg TS]
- c_{C} B, c_{CF}: Kohlenstoffgehalt der Fraktion Biogen und Fossil [kg C/kg aschefrei]
- c_{CA}: Kohlenstoffgehalt des Abfalls [kg C/kg FS]
- c_{C Erdgas}: Kohlenstoffgehalt von Erdgas bzw. Erdöl [kg C/kg]
- c_{H Erdgas}: Wasserstoffgehalt von Erdgas bzw. Erdöl [kg C/kg]
- c_{H B}, c_{HF}: Wasserstoffgehalt der Fraktion Biogen und Fossil [kg H/kg aschefrei]
- c_{OB}, c_{OF}: Sauerstoffgehalt der Fraktion Biogen und Fossil [kg O/kg aschefrei]
- c_{N B}, c_{N F}: Stickstoffgehalt der Fraktion Biogen und Fossil [kg N/kg aschefrei]
- c_{S B}, c_{S F}: Schwefelgehalt der Fraktion Biogen und Fossil [kg S/kg aschefrei]
- V_{R}: Reingasmenge, trocken unter Normalbedingungen [Nm³]
- T_{C,R}: Kohlenstoff-Transferkoeffizient ins Rauchgas [-], = 0,982±0,0025 (z.B.: Morf et al., 1997)
- C_{CO2,R}: Kohlendioxidkonzentration im Reingas, trocken, bezogen auf Normalbedingungen [kg/Nm³]
- H_{u,A,B}: Heizwert des Abfalls nach Boie [MJ/kg FS]
- H_{u,A}: Heizwert des Abfalls (korrigiert nach Kost (2001)) [MJ/kg FS]
- H_{u,Erdgas}: Heizwert von Erdgas bzw. Erdöl [MJ/kg]
- T_{Luft}: Außentemperatur [°C]
- p_{Luft}: Luftdruck(außen) [hPa]
- p_{N}: Luftdruck unter Normalbedingungen = 101,3 [hPa]
- a_{PI-SL}: absolute Feuchte der Primär- und Sekundärluft [kg H₂0/m³]
- a_{N,PL-SL}: absolute Feuchte der Primär- und Sekundärluft bezogen auf Normalbedingungen [kg H₂0/Nm³]
- RF: relative Luftfeuchte [%]
- e: Wasserdampfpartialdruck [hPa] der Luft
- m_{mol,H2O}: Molare Masse von Wasser = 18 g/mol
- R: universelle Gaskonstante = 8,314472 J/(mol*K)
- V_{PL-SL}: Menge an Primär- und Sekundärluft [Nm³]
- m_{H2O,PL-SL}: Wasserinput über die Primär- und Sekundärluft [kg]
- m_{H2O,Müll,H}: Wasserinput über die Wasserstoffkonzentration im Müll [kg]
- m_{H2O,Erdgas}: Wasserinput über die Wasserstoffkonzentration in den Zusatzbrennstoffen [kg]
- T: Temperatur der Primär- und Sekundärluft [°C]
- T_{Wäscher}: Temperatur im Wäscher 1 [°C]
- P_{Wäscher}: Druck im Wäscher 1 [hPa]
- p_{N}: Luftdruck unter Normalbedingungen = 101,3 [hPa]
- a_{Wäscher}: absolute Feuchte des Rohgases 2 im Wäscher 1 [kg H₂O/m³]
- a_{N,Rohgas2}: absolute Feuchte des Rohgases 2 bezogen auf Normalbedingungen [kg H₂0/Nm³]
- pₐ: Sättigungsdampfdruck [hPa]
- m_{H2O,Rohgas2}: Wasseroutput über das Rohgas 2 [kg]
- WD: Wasserdampfmenge [kg]
- h_{T}": Enthalpie des Wasserdampfs im Kessel vor der Turbine [MJ/kg]
- h_{K}': Enthalpie des Kesselwassers im Kondensator bzw. des Speisewassers für den Dampfkessel [MJ/kg]
- η: Wirkungsgrad des Dampfkessels = 0,83 ± 0,04 (Wachter, 2004)
- Δ_{O2+CO2,g}: Differenz aus O₂-Verbrauch und CO₂-Produktion des behandelten Abfalls [mol/kg TS]
- O₂V: Sauerstoffumsatz [mol/kg FS]
- c_{O2,L}: Sallerstoffkonzentration in der Primär- und Sekundärluft [Vol-%, trocken] (=20,946 %)
- c_{CO2,L}: Kohlendioxidkonzentration in der Primär- und Sekundärluft [Vol-%, trocken] (=0,034 %)
- c_{O2,R}: Sauerstoffkonzentration im Reingas [Vol-%, trocken]
- c_{CO2,R}: Kohlendioxidkonzentration im Reingas [Vol-%, trocken]
- mᵥ: Molvolumen [Nm³/mol] (=0,0224 Nm³/mol)

**Abkürzungen**
- MW oder Mittelw.: Mittelwert
- SD oder Stdabw: Standardabweichung

### Berechnung der stofflichen Zusammensetzung von Sortierfraktionen

### Stoffliche Zusammensetzung von Hygienewaren

**Tabelle 21 Stoffliche Zusammensetzung von Hygienewaren**

| **Zusammensetzung** | | **Gruppe** | **C-Gehalt** | **H-Gehalt** | **S-Gehalt** | **N-Gehalt** | **O-Gehalt** |
|---|---|---|---|---|---|---|---|
| | **[m%]** | | [mg/kg Aschefrei] | | | | |
| Zellstoff | 43% | biogen | 444 | 62 | - | - | 494 |
| Polymer (SAP) | 27% | fossil | 500 | 56 | - | - | 444 |
| Polyethylen | 10% | | 857 | 142 | - | - | 0 |
| Polypropylen | 13% | | 857 | 142 | - | - | 0 |
| Klebstoff, Klebebänder | 7% | | 350^{#} | 100^{#} | - | - | 300^{#} |
| ***Summe*** | *100%* | | | | | | |
| ***Biogen*** | ***43 %*** | ***63,8*** | ***444*** | ***62*** | ***2*** | ***8**** | ***494*** |
| ***Fossil*** | ***57 %*** | ***36,2*** | ***625*** | ***96*** | ***2*** | ***8**** | ***247*** |

| | | | | | | | |
|---|---|---|---|---|---|---|---|
| *^{#} eigene Annahmen* ** aufgrund fehlender Literaturdaten wurden Analyseergebnisse von Kost (2001) übernommen* | | | | | | | |

### Stoffliche Zusammensetzung von Textilien

**Tabelle 22 Stoffliche Zusammensetzung von Textilien**

| **Zusammensetzung** | **Gruppe** | **Anteil an der Weltproduktion** | **C-Gehalt** | **H-Gehalt** | **S-Gehalt** | **N-Gehalt** | **O-Gehalt** |
|---|---|---|---|---|---|---|---|
| | | [m%] | [mg/kg Aschefrei] | | | | |
| Baumwolle | biogen | 50,9% | 444 | 62 | - | - | 494 |
| Wolle | | 4,6% | 482 | 63 | 71 | 162 | 221 |
| Viscose | | 8,3% | 444 | 62 | - | - | 494 |
| Polyamide | fossil | 8,8% | 637 | 97 | - | 124 | 142 |
| Polyester | | 19,7% | 625 | 42 | - | 0 | 333 |
| Palyacrylnitril | | 5,3% | 647 | 59 | - | 223 | 71 |
| Polyproylen | | 2,3% | 857 | 143 | - | - | 0 |
| *Summe* | | *100%* | | | | | |
| ***Biogen*** | | ***63,8*** | ***447*** | ***62*** | ***4**** | ***12*** | ***474*** |
| ***Fossil*** | | ***36,2*** | ***646*** | ***64*** | ***4**** | ***63*** | ***227*** |

| | | | | | | | |
|---|---|---|---|---|---|---|---|
| ** aufgrund fehlender Literaturdaten wurden Analyseergebnisse von Kost (2001) übernommen* | | | | | | | |

### Betriebsdaten der untersuchten MVA im Zeitraum Oktober 2004 bis September 2005

**Tabelle 23 meteorologische Daten am Standort, Luftmengen sowie monatliche Wasseflüsse der untersuchten MVA**

| | **Okt. 04** | **Nov. 04** | **Dez. 04** | **Jän. 05** | **Feb. 05** | **Mär. 05** | **Apr. 05** | **Mai. 05** | **Jun. 05** | **Jul. 05** | **Aug. 05** | **Sep. 05** |
|---|---|---|---|---|---|---|---|---|---|---|---|---|
| Primärluft [1.000 m³/Mo] | 29.094 | 32.428 | 29.622 | 28.416 | 25.241 | 30.500 | 29.502 | 30.898 | 30.027 | 20.389 | 28.217 | 30.900 |
| Sekundärluft [1.000 m³/Mo] | 9.841 | 11.648 | 11.962 | 9.639 | 7.755 | 8.579 | 9.362 | 9.841 | 9.768 | 6.223 | 8.244 | 5.706 |
| Reingas, trocken [1.000 Nm³/Mo] | 57.164 | 56.933 | 60.534 | 57.906 | 49.785 | 57.424 | 55.536 | 57.197 | 55.980 | 38.692 | 57.599 | 56.964 |
| mittl. Lufttemp. [°C] | 6,7 | 5,2 | 5,0 | -0,1 | 2,2 | 3,8 | 10,5 | 13,0 | 16,7 | 18,7 | 19,5 | 9,7 |
| mittl. Luftfeuchte [%] | 72,5 | 82,5 | 80,1 | 75,2 | 67,7 | 68,8 | 64,3 | 62,2 | 58,7 | 58,5 | 55,3 | 67,5 |
| mittl. Luftdruck [mbar] | 978 | 984 | 950 | 975 | 982 | 984 | 976 | 977 | 981 | 980 | 977 | 977 |
| Temperatur im Wäscher 1 [°C] | 58,3 | 58,2 | 54,6 | 56,4 | 59,9 | 56,0 | 59,9 | 60,1 | 61,9 | 62,7 | 63,2 | 58,0 |
| Druck im Wäscher 1 [mbar] | 977 | 982 | 949 | 974 | 980 | 982 | 975 | 976 | 980 | 979 | 976 | 976 |
| Wassergehalt von Primär- und Sekundärluft [kg/Nm³] | 0,0058 | 0,0060 | 0,0059 | 0,0038 | 0,0040 | 0,0045 | 0,0067 | 0,0077 | 0,0092 | 0,010 | 0,010 | 0,0069 |
| Wassergehalt im Reingas (im Wäscher 1 - gesättigt) [kg/Nm³] | 0,149 | 0,151 | 0,150 | 0,131 | 0,138 | 0,163 | 0,135 | 0,164 | 0,165 | 0,178 | 0,185 | 0,190 |
| Wasserimport über Primär- und Sekundärluft [t/Mo] | 226 | 264 | 245 | 145 | 132 | 176 | 260 | 314 | 366 | 266 | 365 | 253 |
| Wasserexport - Wäscher 1 [t/Mo] | 2.108 | 2.085 | 1.889 | 1.910 | 1.599 | 1.846 | 1.792 | 1.409 | 1.432 | 1.191 | 1.835 | 1.072 |
| Reingas [t/Mo] | 9.377 | 9.418 | 10.898 | 10.387 | 9.148 | 10.174 | 10.329 | 10.620 | 11.520 | 8.345 | 11.741 | 5.224 |

## Patentansprüche

1. Verfahren zur Ermittlung der Anteile biogener und fossiler Energieträger sowie biogener und fossiler Kohlendioxidemissionen beim Betrieb von Verbrennungsanlagen, **dadurch gekennzeichnet, dass** die Ermittlung der Anteile durch die Bestimmung von zumindest drei der folgenden Bilanzen erfolgt: der Massenbilanz, der Aschenbilanz, der Kohlenstoffbilanz, der Energiebilanz, der Sauerstoffumsatzbilanz, der Bilanz aus Sauerstoffverbrauch und Kohlendioxidproduktion und der Wasserbilanz.

2. Verfahren nach Anspruch 1, **dadurch gekennzeichnet, dass** die Ermittlung der Anteile durch die Bestimmung von zumindest vier Bilanzen erfolgt.

3. Verfahren nach einem der Ansprüche 1 oder 2, **dadurch gekennzeichnet, dass** die Ermittlung der Anteile durch die Bestimmung der Massenbilanz, der Aschenbilanz, der Kohlenstoffbilanz, der Energiebilanz, der Sauerstoffumsatzbilanz, der Bilanz aus Sauerstoffverbrauch und Kohlendioxidproduktion erfolgt.

4. Verfahren nach einem der Ansprüche 1 bis 3, **dadurch gekennzeichnet, dass** die Anteile mittels Ausgleichsrechnung ermittelt werden.

5. Verfahren nach Anspruch 1 bis 4, **dadurch gekennzeichnet, dass** die Anteile mittels Monte Carlo Simulation ermittelt werden.

6. Verfahren nach einem der voranstehenden Ansprüche, **dadurch gekennzeichnet, dass** bei einem Einsatz von Abfall als Energieträger die unterschiedlichen Materialien des Abfalls in drei Stoffgruppen: Inert wie Glas, Metall,
inklusive seines Wasseranteil; Biogen wie Papier, Küchenabfälle, inklusive seines Asche- und Wasseranteils; und Fossil wie Kunststoff, Kunststoffanteile in Textilien, inklusive seines Asche- und Wasseranteils; unterteilt werden.

7. Verfahren nach Anspruch 6, **dadurch gekennzeichnet, dass** die Elementarzusammensetzungen der drei Stoffgruppen bestimmt und der Berechnung zugrunde gelegt werden.

8. Verfahren nach einem der Ansprüche 2 bis 5, **dadurch gekennzeichnet, dass** bei einem Einsatz von Abfall als Energieträger die unterschiedlichen Materialien des Abfalls in vier Stoffgruppen: Inert wie Glas, Metall,
exklusive seines Wasseranteils; Biogen wie Papier, Küchenabfälle, exklusive seines Asche- und Wasseranteils; Fossil wie Kunststoff, Kunststoffanteile in Textilien exklusive seines Asche- und Wasseranteils; und Wasser unterteilt werden.

9. Verfahren nach Anspruch 8, **dadurch gekennzeichnet, dass** die mittleren Elementarzusammensetzungen der biogenen und der fossilen Stoffgruppen bestimmt und der Berechnung zugrunde gelegt werden.

## Claims

1. Method for determining the proportion of biogenic and fossil energy carriers and of biogenic and fossil carbon dioxide emissions in the operation of combustion systems, **characterised in that** determining the proportion is carried out by identifying at least three of the following balances: mass balance, ash balance, carbon balance, energy balance, oxygen consumption balance, oxygen consumption and carbon dioxide production balance and water balance.

2. Method according to claim 1, **characterised in that** determining the proportion is carried out by identifying at least four balances.

3. Method according to one of claims 1 or 2, **characterised in that** determining the proportion is carried out by identifying the mass balance, ash balance, carbon balance, energy balance, oxygen consumption balance and oxygen consumption and carbon dioxide production balance.

4. Method according to one of claims 1 to 3, **characterised in that** the proportion is determined by means of regression calculation.

5. Method according to claims 1 to 4, **characterised in that** the proportion is determined by means of Monte Carlo Simulation.

6. Method according to one of the preceding claims, **characterised in that**, when using waste as the energy source, the different waste materials are subdivided into three substance groups: Inert, such as glass, metal, including its water proportion; Biogenic, such as a paper, kitchen waste, including its ash and water proportion; and Fossil, such as plastic, plastic proportions in textiles, including its ash and water proportion.

7. Method according to claim 6, **characterised in that** the elemental compositions of the three substance groups are determined and are based on the calculation.

8. Method according to one of claims 2 to 5, **characterised in that**, when using waste as the energy source, the different waste materials are subdivided into four substance groups: Inert, such as glass, metal, not including its water proportion; Biogenic, such as a paper, kitchen waste, not including its ash and water proportion; Fossil, such as plastic, plastic proportions in textiles, not including its ash and water proportion; and water.

9. Method according to claim 8, **characterised in that** the average elementary compositions of the biogenic and fossil substance groups are determined and are based on the calculation.

## Revendications

1. Procédé de détermination des parts de sources d'énergie blogènes et fossiles ainsi que des émissions blogènes et fossiles de dioxyde de carbone dans le fonctionnement d'incinérateurs, **caractérisé en ce que** la détermination des parts est réalisée par la détermination d'au moins trois des bilans suivants : le bilan massique, le bilan des cendres, le bilan du carbone, le bilan énergétique, le bilan de réaction de l'oxygène, le bilan de la consommation d'oxygène et de la production de dioxyde de carbone et le bilan de l'eau.

2. Procédé selon la revendication 1, **caractérisé en ce que** la détermination des parts est réalisée par la détermination d'au moins quatre bilans.

3. Procédé selon l'une quelconque des revendications 1 ou 2, **caractérisé en ce que** la détermination des parts est réalisée par la détermination du bilan massique, du bilan des cendres, du bilan du carbone, du bilan énergétique, du bilan de réaction de l'oxygène, du bilan de la consommation d'oxygène et de la production de dioxyde de carbone.

4. Procédé selon l'une quelconque des revendications 1 à 3, **caractérisé en ce que** les parts sont déterminées à l'aide d'un calcul d'équilibre.

5. Procédé selon l'une des revendications 1 à 4, **caractérisé en ce que** les parts sont déterminées à l'aide d'une simulation de Monte Carlo.

6. Procédé selon l'une quelconque des revendications précédentes, **caractérisé en ce que** lors de l'utilisation de déchets comme sources d'énergie, les différentes matières des déchets sont divisées en trois groupes de matiéres : les matières Inertes comme le verre, le métal comprenant leur part en eau ; les matières biogènes comme le papier, les déchets de cuisine, comprenant leur part en cendres et en eau ; les matières fossiles comme les matières plastiques, les parts synthétiques dans des textiles, comprenant leur part en cendres et en eau.

7. Procédé selon la revendication 6, **caractérisé en ce que** les compositions élémentaires des trois groupes de matières sont déterminées et sont à la base du calcul.

8. Procédé selon l'une quelconque des revendications 2 à 5, **caractérisé en ce que** lors de l'utilisation de déchets comme sources d'énergie, les différentes matières des déchets sont divisées en quatre groupes de matières : les matières Inertes comme le verre, le métal, à l'exclusion de leur part en eau ; les matières biogènes comme le papier, les déchets de cuisine, à l'exclusion de leur part en cendres et en eau ; les matières fossiles comme les matières plastiques, les parts synthétiques dans des textiles à l'exclusion de leur part en cendres et en eau ; et l'eau.

9. Procédé selon la revendication 8, **caractérisé en ce que** les compositions élémentaires moyennes des groupes de matières biogènes et fossiles sont déterminées et sont à la base du calcul.
